# EUROPEAN PATENT SPECIFICATION

(11) **EP 4 343 330 B1**
(45) Date of publication and mention of the grant of the patent: **18.11.2026**
(21) Application number: 22804921.9
(22) Date of filing: 13.05.2022
(51) Int. Cl.: G01N 33/68

(54) **MARKER FOR DIAGNOSING NON-ALCOHOLIC FATTY LIVER DISEASE**
MARKER ZUR DIAGNOSE VON NICHTALKOHOLISCHER FETTLEBERERKRANKUNG
MARQUEUR POUR DIAGNOSTIQUER UNE STÉATOSE HÉPATIQUE NON ALCOOLIQUE

(30) Priority: 21.05.2021 KR 20210065761; 21.05.2021 KR 20210065760; 16.06.2021 KR 20210078025
(43) Date of publication of application: 27.03.2024
(73) Proprietor: Brexogen Inc., Seoul 05855 (KR)
(72) Inventor: KIM, Sue, Seoul 05649 (KR); KIM, Ji Min, Seoul 05837 (KR)
(74) Representative: Plougmann Vingtoft a/s
(86) International application number: PCT/KR2022/006924
(87) International publication number: WO 2022/245066

(56) References cited:
- WO-A1-2018/037229
- WO-A1-2020/021058
- KR-A- 20210 024 417
- US-A1- 2006 135 420
- US-A1- 2008 090 223
- US-A1- 2020 199 575
- JEPPESEN DENNIS K ET AL: "Reassessment of Exosome Composition", CELL, vol. 177, no. 2, 2019, pages 428, XP085654993, ISSN: 0092-8674, DOI: 10.1016/J.CELL.2019.02.029
- HOLCAR MARIJA ET AL: "Enrichment of plasma extracellular vesicles for reliable quantification of their size and concentration for biomarker discovery", vol. 10, no. 1, 7 December 2020 (2020-12-07), US, XP093194331, ISSN: 2045-2322, Retrieved from the Internet <URL:https://www.nature.com/articles/s41598-020-78422-y> DOI: 10.1038/s41598-020-78422-y
- SUN, Y. ET AL.: "Lipid profile characterization and lipoprotein comparison of extracellular vesicles from human plasma and serum.", METABOLITES, vol. 9, no. 259, November 2019 (2019-11-01), pages 1 - 15, XP093008140
- KARAVIA ELENI A, PAPACHRISTOU DIONYSIOS J, LIOPETA KASSIANI, TRIANTAPHYLLIDOU IRENE-EVA, DIMITRAKOPOULOS ODYSSEFS, KYPREOS KYRIAKO: "Apolipoprotein AI modulates processes associated with diet-induced nonalcoholic fatty liver disease in mice.", MOLECULAR MEDICINE, vol. 18, 2012, pages 901 - 912, XP093008145

## Description

### Technical Field

The present disclosure relates to a A method for providing information for diagnosis of nonalcoholic fatty liver disease (NAFLD), the method comprising a measuring step of measuring a level of exosome-derived (i.e. exosomal) ApoA-1 protein in a biological sample isolated from a subject that has developed or is suspected of onset of nonalcoholic fatty liver disease (NAFLD) therein; wherein a comparison step of comparing protein level measurements between the subject and a normal control; and wherein a determining step of determining the onset of nonalcoholic fatty liver disease in the subject when the measurement in the biological sample is lower than that in a sample from a normal control.

### Background Art

Mesenchymal stem cells are stromal cells that can differentiate into various cells including osteoblast cells, chondrocytes, muscular cells, adipocytes, etc. Being capable of differentiating into various connective tissues such as cartilages, bone tissues, ligaments, marrow stromal cells, etc., mesenchymal stem cells have been studied for use in treating various diseases including arthritis, soft tissue defects caused by trauma and burn, and so on.

Meanwhile, non-alcoholic steatosis is characterized by the accumulation of triglycerides in hepatocytes without excessive intake of alcohol. Non-alcoholic steatosis continues to increase due to excessive nutrition associated with high-fat and high-carbohydrate intake in modern people. For obesity and diabetes, non-alcoholic steatosis is frequently observed, but various factors are known to be relevant to non-alcoholic steatosis. It is reported that 80% of adults with non-alcoholic steatosis develop metabolic diseases such as insulin-resistant diabetes and heart disease.

Non-alcoholic steatosis is classified into non-alcoholic simple steatosis and non-alcoholic steatohepatitis (NASH) with inflammation, and if left untreated for a long time, can be developed into serious liver diseases such as hepatitis, liver fibrosis, cirrhosis, etc. Non-alcoholic steatosis is characterized by the accumulation of fat (fat infiltration) in hepatocytes.

Non-alcoholic simple steatosis can progress into non-alcoholic steatohepatitis. In non-alcoholic steatohepatitis, the fat accumulation is associated with varying degrees of inflammation and scarring of the liver, and in many cases insulin resistance, dyslipidemia, and hypertension. Non-alcoholic steatohepatitis most often occurs in people with excess body weight, excess blood cholesterol and triglyceride levels, and/or insulin resistance.

Jeppesen et al., 2019, Cell 177, 428-445 April 4, 2019, discloses e.g. immunoblots of high-resolution density gradient-purified sEV (small extracellular vesicles) and NV (non-vesicular) samples isolated from plasma of three normal individuals and states that in human plasma, Ago2 and HDL (ApoA1) were present in gradient-purified NV pool, but absent from the sEV pool.

Holcar M. et al., Scientific Reports, 2020, Dec 7;10(1), discloses e.g. that in order to support TEM (transmission electron microscopy) results, concentration of lipoproteins in SEC (size exclusion chromatography) and sUC (sucrose cushion ultracentrifugation) EV-enriched samples was measured by ELISAs specific for Apolipoprotein A1 (ApoA1) and Apolipoprotein B100 (ApoB100), characteristic for HDLs and LDLs, IDLs or VLDLs, respectively.

WO2020/021058 discloses e.g. an in vitro method for diagnosing the presence of liver steatosis in a subject based on *inter alia* circulating biochemical markers, such as ApoA1.

However, despite the increase in non-alcoholic steatohepatitis patients along with the recent increase in obese population, the development of a safe and long-term usable treatment for non-alcoholic steatohepatitis still remains insignificant, and the development of preparations or kits for diagnosing non-alcoholic steatohepatitis is not satisfactory. Therefore, there is a growing need for the development of a diagnostic composition or kit suitable for diagnosing non-alcoholic steatohepatitis, a chronic disease, as well as a prognostic composition after administration of a non-alcoholic steatohepatitis treatment.

### Detailed Description of the Invention

### Technical Problem

Intensive and thorough research conducted by the present inventors has resulted in the finding that nonalcoholic fatty liver disease (NAFLD) can be diagnosed quickly and accurately through exosome-derived ApoA-1 (hereinafter referred to as exosomal ApoA-1) protein and this protein can be used as a diagnostic marker for non-alcoholic fatty liver disease.
Another aspect of the present disclosure is to provide a method for providing information for diagnosis of non-alcoholic fatty liver disease.

### Technical Solution

The present inventors succeeded in accurately diagnosing and prognosing nonalcoholic fatty liver disease

In the present disclosure, it was discovered that an agent for measuring a level of exosome-derived ApoA-1 (exosomal ApoA-1) protein can provide more accurate information about the diagnosis and prognosis of nonalcoholic fatty liver disease, compared with conventional methods.

Below, a more detail description will be given of the present disclosure.

As used herein, the term "marker" refers to a substance capable of diagnosing nonalcoholic fatty liver disease by distinguishing an individual affected therewith from a normal individual, and the marker may be any organic biological molecule that quantitatively increases or decreases in individuals with nonalcoholic fatty liver disease, such as polypeptides, proteins or nucleic acids, lipids, glycolipids, glycoproteins, sugars, etc. In the present invention, the marker may be, for example, a protein the level of which is increased in an individual affected with nonalcoholic fatty liver disease, but is not limited thereto.

As used herein, the term "exosome" refers to a membrane vesicle that is extracellularly secreted from a cell or has a membrane structure composed of a lipid-bilayer present in the cell, and exosomes are found in the body fluid of almost all eukaryotes. Exosomes are about 30 to 1000 nm in diameter. Cells release exosomes directly from cell membranes when multivesicular bodies are fused to cell membranes. It is well known that exosomes play a functional role in mediating coagulation, cell-cell communication, and cellular immunity by transporting intracellular biomolecules, such as proteins, bioactive lipids, and RNA (miRNA).

In the present disclosure, the exosomes are intended to encompass microvesicles. CD63 and CD81 are known as marker proteins of exosomes. In addition, various proteins including, for example, cell surface receptors such as EGFR, signaling-related molecules, cell adhesion-related proteins, MSC-associated antigens, heat shock proteins, and vesiculation-related Alix are found in exosomes.

As used herein, the term "ApoA-1 protein" refers to a protein encoded by ApoA-1 gene, known to play a specific role as a main protein component of HDL particles in lipid metabolism as the main protein component of HDL particles. Although ApoA-1 is frequently used as a biomarker for the prediction of cardiovascular diseases, no known applications exist for exosomal ApoA-1 protein as a biomarker. The present inventors have confirmed that the onset of nonalcoholic fatty liver disease in an individual, or the suspicion thereof, can be accurately diagnosed by measuring the protein levels of exosomal ApoA-1 in the individual's plasma.

Nonalcoholic fatty liver disease is one of the most common liver diseases and is known to be closely related to type 2 diabetes, obesity, and metabolic syndrome. As used herein, the term "nonalcoholic fatty liver disease" is intended to encompass a wide range of liver diseases from simple steatosis to non-alcoholic steatohepatitis (NASH) and further to cirrhosis.

As used herein, the term 'diagnosis' or 'diagnosing' means identifying the presence or characteristic of a certain pathological condition. For the sake of the present disclosure, the term "diagnosis" refers to determining the onset of nonalcoholic fatty liver disease.

In an embodiment of the present disclosure, the nonalcoholic fatty liver disease may be non-alcoholic steatohepatitis (NASH).

As used herein, the term "non-alcoholic steatohepatitis" refers to a form of nonalcoholic fatty liver disease (NAFLD), a progressive liver disease characterized by fatty liver with inflammation or fibrosis, which is a precursor disease leading eventually to cirrhosis or liver cancer.

As used herein, the term "agent for measuring a protein level" refers to an agent used in a method for measuring the level of proteins included in a sample. The agent for measuring a protein level may include protein-detecting agents known in the art, for example, antibodies used in the methods such as western blotting, ELISA (enzyme linked immunosorbent assay), radioimmunoassay (RIA), radioimmunodiffusion, Ouchterlony immunodiffusion, rocket immunoelectrophoresis, immunohistochemical staining, immunoprecipitation assay, complement fixation assay, immunofluorescence, immunochromatography, FACS (fluorescenceactivated cell sorter analysis), and protein chip technology assay, but with no limitations thereto.

In the present disclosure, the agent for measuring a protein level may include an antibody or aptamer specific for a protein of interest.

In the present disclosure, the antibody may be at least one selected from the group consisting of monoclonal antibodies, polyclonal antibodies, antibody fragments, and recombinant antibodies.

As used herein, the term "antibody" refers to a protein molecule that may bind specifically to the antigenic site of a protein or peptide molecule. This antibody may be produced by cloning each gene into an expression vector according to a conventional method to obtain a protein encoded by the marker gene and producing the antibody from the obtained protein according to a conventional method. The form of the antibody is not specifically limited, and polyclonal antibodies, monoclonal antibodies, or portions thereof which have antigen binding ability are included in the antibodies of the present invention, and all immunoglobulin antibodies, a special antibody such as humanized antibody, etc. may also be included in the antibodies of the present invention. Furthermore, the antibody includes not only a complete antibody having two full-length light chains and two full-length heavy chains, but also a functional fragment of the antibody molecule. The expression "functional fragment of the antibody molecule" refers to a fragment having at least antigen binding ability, and examples of the functional fragment include Fab, F(ab'), F(ab')2, Fv, etc.

The aptamer is a single-stranded oligonucleotide, and refers to a nucleic acid molecule having binding activity to a given target molecule. The aptamer may have various three-dimensional structures depending on its nucleotide sequence, and may have high affinity for a specific substance, as in an antigen-antibody reaction. The aptamer may inhibit activity of a given target molecule by binding to the given target molecule.

The aptamer of the present disclosure may be RNA, DNA, a modified nucleic acid, or a mixture thereof, and its shape may be in a linear or circular form, but is not limited thereto. The aptamer having binding activity for a given protein may be easily prepared by those skilled in the art according to a known method with reference to each nucleotide sequence.

Another aspect of the present disclosure is drawn to a method for providing information for diagnosis of nonalcoholic fatty liver disease in a subject, the method including a step of measuring a level of exosomal ApoA-1 protein in a biological sample isolated from the subject that has developed or is suspected of the onset of nonalcoholic fatty liver disease.

In an embodiment of the present disclosure, the method may further include a step of comparing protein level measurements between the subject and a normal control.

In an embodiment of the present disclosure, the measuring step may include contacting the sample with an antibody or aptamer binding specifically to ApoA-1 protein.

As used herein, the term "subject" may refer to all kinds of animals including humans, which have developed non-alcoholic fatty liver disease or are at risk for the disease. The animals may be mammals such as cattle, horses, sheep, pigs, goats, camels, antelopes, dogs, and cats, which are in need of treatment for similar symptoms, as well as humans, but are not limited thereto.

As used herein, the term "normal control" refers to a subject that has not developed nonalcoholic fatty liver disease or that is not suspected of having the disease.

As used herein, the term "sample" refers to the direct target to be measured for the expression level of the exosomal ApoA-1 protein, which is separated from patients who have developed nonalcoholic fatty liver disease.

In an embodiment of the present disclosure, the sample may be at least one selected from the group consisting of tissues, cells, whole blood, sera, and plasma, each isolated from a subject.

In an embodiment of the present disclosure, the method may further include a step of determining the onset of nonalcoholic fatty liver disease in the subject when the measurement in the biological sample is lower than that in a sample from a normal control.

As used herein, the term "therapeutic candidate" refers to a substance that increases the level of ApoA-1 protein in exosomes measured in a subject's sample, and has the potential for treating nonalcoholic fatty liver disease. The term is intended to encompass, without limitation, oligonucleotides, proteins, and compounds.

The term "oligonucleotide", as used herein, refers to a polymer formed by several to tens of nucleotides polymerized through phosphodiester bonds.

In the comparison step of the screening method according to the present disclosure, the level of exosomal ApoA-1 protein in the biological sample separated from the subject significantly increases compared to the control group, this condition signifies that the candidate has the potential as a therapeutic agent for nonalcoholic fatty liver disease.

As used herein, the term "therapeutic agent for nonalcoholic fatty liver disease" refers to any substance that can treat nonalcoholic fatty liver disease or reduce or alleviate the severity or related parameters of nonalcoholic fatty liver disease. For example, the present inventors found that exosomes isolated from induced pluripotent stem cell (iPSC)-derived mesenchymal stem cells (MSC) are superior to existing clinical therapeutics in terms of therapeutic, palliative, and alleviative effects for nonalcoholic fatty liver disease.

As used herein, the term "prognosis" refers to the action of predicting the course of a disease and the result of death or survival in advance. More specifically, prognosis or prognostic prediction can be interpreted as any act of predicting the course of a disease before and after treatment, considering the patient's comprehensive physiological or environmental state which can vary the course of the disease. In the context of the present disclosure, prognosis can be interpreted as the action of predicting the course and cure of nonalcoholic fatty liver disease before and after treatment, and predicting the disease-free survival rate or survival rate of nonalcoholic fatty liver disease patients.

For example, predicting a "good prognosis" signifies a high survival rate for nonalcoholic fatty liver disease patients regardless of treatment, or an improved level of indicators related to fatty liver disease, implying a high likelihood of the patient being cured. Predicting a "poor prognosis" means that the survival rate of patients after treatment of nonalcoholic fatty liver disease is low, or the level of indicators related to fatty liver disease has worsened.

The present inventors have confirmed that measuring the level of exosomal ApoA-1 protein is able to more accurately assess the amelioration or aggravation of nonalcoholic fatty liver disease compared to measuring the level of ApoA-1 protein in serum, which is used in existing clinical practice.

As used herein, the term "stem cell" refers to an undifferentiated cell that has the ability to self-replicate and differentiate into two or more different types of cells. The stem cells in the disclosure may be either autologous or allogeneic in origin.

The term "induced pluripotent stem cells", as used herein, refers to cells that have been reprogrammed back into an undifferentiated state of pluripotency by inducing dedifferentiation in already differentiated cells such as somatic cells.

The dedifferentiation can be induced by introducing and expressing specific genes (e.g., Sox2, c-Myc, Klf4, Oct-4, etc.) or injecting dedifferentiation inducing proteins expressed in the cells having the specific genes introduced thereinto.

Pluripotency is defined as the potential to differentiate into tissues or organs of any origin of the three germ layers endoderm, mesoderm, and ectoderm. As used herein, the term "mesenchymal stem cells" refers to pluripotent stem cells capable of differentiating into various types of cells including osteoblasts, chondrocytes, myocytes, lipocytes, etc. The mesenchymal stem cells may be usually bone marrow-derived mesenchymal stem cells, but may be derived from umbilical cord, umbilical cord blood, adipose tissues, amniotic fluid, or molar tooth buds. Mesenchymal stem cells are also called stromal cells.

The "induced pluripotent stem cell", as used herein, refers to cells which have been induced to have pluripotency by artificial dedifferentiation from differentiated cells and is also known as dedifferentiated stem cells.

In the present disclosure, the induced pluripotent stem cell-derived mesenchymal stem cells may be derived from a progenitor of induced pluripotent stem cell-derived mesenchymal stem cells which does not express SSEA-4 (stage-specific embryonic antigen 4) protein.

As used herein, the term "induced pluripotent stem cell-derived mesenchymal stem cell progenitor" refers to cells in the stage just before induced pluripotent stem cells are not differentiated completely into mesenchymal stem cells, which are a kind of induced pluripotent stem cell-derived mesenchymal stem cells, and means cells that do not express SSEA-4 protein and become complete mesenchymal stem cells through further culturing.

In the present disclosure, the induced pluripotent stem cell-derived mesenchymal stem cells may be those differentiated from progenitor cells of induced pluripotent stem cell-derived mesenchymal stem cells that do not express SSEA-4 protein.

The artificial dedifferentiation process is performed by introduction of a dedifferentiation factor through viral mediation using retrovirus, lentivirus, and Sendai virus or through non-viral mediation using a non-viral vector, a protein, and a cell extract, or by a stem cell extract, a compound, etc.

The induced pluripotent stem cells have almost the same traits as embryonic stem cells, specifically are similar in cell morphology and gene and protein expression patterns, exhibit pluripotency in vitro and in vivo, and develop teratoma. When the induced pluripotent stem cells were inserted into mouse blastocysts, chimeric mice were generated with germ line transmission observed therein.

The induced pluripotent stem cells of the present disclosure are intended to encompass induced pluripotent stem cells derived from all mammals such as humans, monkeys, pigs, horses, cows, sheep, dogs, cats, mice, rabbits, and so on, with preference for human-derived induced pluripotent stem cells.

In addition, the somatic cells from which the induced pluripotent stem cells of the present disclosure are dedifferentiated may be somatic cells derived from umbilical cord, umbilical cord blood, bone marrow, fat, muscle, nerve, skin, amniotic membrane, amniotic fluid, or placenta, but with no limitations thereto.

In the present disclosure, exosomes isolated from the induced pluripotent stem cell-derived mesenchymal stem cells refer to exosomes found within the induced pluripotent stem cell-derived mesenchymal stem cells (BxC) or released from BxC.

As used herein, the term "comprising as an active ingredient" refers to comprising exosomes isolated from the induced pluripotent stem cell-derived mesenchymal stem cells in an amount sufficient to attain activity to prevent or treat non-alcoholic fatty liver disease.

As used herein, the term "pretreatment" refers to a process of contacting progenitor cells of induced pluripotent stem cell-derived mesenchymal stem cells with a cell culture medium containing a pretreatment material during culturing of the progenitor cells.

Another aspect of the present disclosure pertains to a method for providing information for prognosis of nonalcoholic fatty liver disease, the method including a measuring step of measuring a level of exosomal ApoA-1 protein in a biological sample isolated from a subject that has been administered a therapeutic agent for nonalcoholic fatty liver disease.

In an embodiment of the present disclosure, the method may further include a comparison step of comparing protein level measurements between the subject and a normal control.

In an embodiment of the present disclosure, the measuring step may include contacting the sample with an antibody or aptamer binding specifically to the protein.

In an embodiment of the present disclosure, the biological sample may be at least one selected from the group consisting of tissues, cells, whole blood, sera, and plasma, each isolated from a subject.

In an embodiment of the present disclosure, the method may be adapted to determine that the risk of developing nonalcoholic fatty liver disease is high or the prognosis of nonalcoholic fatty liver disease is poor, if the level of exosome-derived ApoA-1 protein measured in the sample isolated from a subject suspected of the onset of nonalcoholic fatty liver disease is lower than the level measured in the sample isolated from a normal control.

In an embodiment of the present disclosure, the method may further include a determination step of determining that the risk of developing nonalcoholic fatty liver disease is high or the prognosis of nonalcoholic fatty liver disease is poor, if the level of exosome-derived ApoA-1 protein measured in the sample isolated from a subject suspected of the onset of nonalcoholic fatty liver disease is lower than the level measured in the sample isolated from a normal control.

In an embodiment of the present disclosure, the nonalcoholic fatty liver disease may be non-alcoholic steatohepatitis (NASH).

### Advantageous Effects

The present disclosure pertains to a method for providing information for diagnosis of nonalcoholic fatty liver disease (NAFLD), the method comprising a measuring step of measuring a level of exosome-derived (i.e. exosomal) ApoA-1 protein in a biological sample isolated from a subject that has developed or is suspected of onset of nonalcoholic fatty liver disease (NAFLD) therein; wherein a comparison step of comparing protein level measurements between the subject and a normal control; and wherein a determining step of determining the onset of nonalcoholic fatty liver disease in the subject when the measurement in the biological sample is lower than that in a sample from a normal control

### Brief Description of the Drawings

FIG. 1 is a heat map exhibiting proteomic signatures in BxC-e (exosomes from unpretreated BxC) and BxC-V37e (exosomes from induced pluripotent stem cell-derived mesenchymal stem cell pretreated with lanifibranor).
FIG. 2 is a plot of drug signatures of BxC-V37e exosomes according to an embodiment of the present disclosure.
FIG. 3 shows a KEGG pathway of the BxC-V37e signature in HepG2 cells, wherein the red characters account for the signature proteins of BxC-V37e, the blue characters for the signature genes upregulated upon BxC-V37e treatment, and the black characters for the targets associated with the signature drugs of BxC-V37e.
FIG. 4 shows photographic images of liver tissues from normal mice and mice with methionine/choline-deficient (MCD) diet-induced NASH that received PBS or BxC-V37e.
FIG. 5 is a graph of serum ALT levels in NASH mice that received PBS or BxC-V37e (normal; n = 6, and MCD-diet; n = 5).
FIG. 6 is a graph of serum AST levels in NASH mice that received PBS or BxC-V37e (normal; n = 6, and MCD-diet; n = 5).
FIG. 7 shows photographic images of hematoxylin and eosin staining in liver tissues from mice administered an MCD diet and PBS or BxC-V37e (GLP-1R agonist used as a positive control, scale bar: 50 µm).
FIG. 8 is a graph of NAFLD activity score (NAS) analyzed using the index of inflammation, hypertrophy, and steatosis scores (data are presented as mean ± SEM, * p <0.05; *** p <0.001).
FIG. 9 shows inhibitory effects of exosomes (BxC-e) isolated from precursor cells (BxC) of induced pluripotent stem cell-derived mesenchymal stem cells.
FIG. 10 is a photographic image of immunoblots (western blots) of exosomal ApoA-1 obtained from sera of NASH mice to which PBS, a GLP-1R agonist, or BxC-V37e has been administered according to an embodiment of the present disclosure.
FIG. 11 is a graph of immunoblots (western blots) of exosomal ApoA-1 obtained from sera of NASH mice to which PBS, a GLP-1R agonist, or BxC-V37e has been administered according to an embodiment of the present disclosure.
FIG. 12 is a plot of human serum exosomal ApoA-1 levels in control (n=20) or obese NAFLD subjects with type 2 diabetes (n=16) or without type 2 diabetes (n=19).
FIGS. 13 to 24 are plots of correlations between levels of exosomal ApoA-1 and metabolic parameters [BMI, ALT, AST, SBP (Systolic blood pressure), DBP (Diastolic blood pressure), and Insulin, HOMA-IR, Hs-CRP, TG, TC, HIS, and NAFLD liver fat score] in human sera according to an embodiment of the present disclosure.
FIGS. 25 to 36 are plots of correlations between levels of exosomal ApoA-1 and metabolic parameters [BMI, ALT, AST, SBP (Systolic blood pressure), DBP (Diastolic blood pressure), Insulin, HOMA-IR, Hs-CRP, TG, TC, HIS, and NAFLD liver fat score].
FIG. 37 is a plot illustrating the correlation between the rate of change in AST levels, a known NASH-related parameter, in serum ApoA-1 and exosome-derived ApoA-1, after administering dulaglutide, a known NASH therapeutic candidate, to MCD-diet mice, compared to the PBS-administered group.
FIG. 38 is a plot illustrating the correlation between the rate of change in hs-CRP levels, a known NASH-related parameter, in serum ApoA-1 and exosome-derived ApoA-1, after administering dulaglutide, a known NASH therapeutic candidate, to MCD-diet mice, compared to the PBS-administered group.
FIG. 39 is a plot illustrating the correlation between the rate of change in ACC1 levels, a known NASH-related parameter, in serum ApoA-1 and exosome-derived ApoA-1, after administering dulaglutide, a known NASH therapeutic candidate, to MCD-diet mice, compared to the PBS-administered group.
FIG. 40 is a plot illustrating the correlation between the rate of change in NRF2 levels, a known NASH-related parameter, in serum ApoA-1 and exosome-derived ApoA-1, after administering dulaglutide, a known NASH therapeutic candidate, to MCD-diet mice, compared to the PBS-administered group.
FIG. 41 is a plot illustrating the correlation between the rate of change in AST levels, a known NASH-related parameter, in serum ApoA-1 and exosome-derived ApoA-1, after administering BxC-V37e to MCD-diet mice, compared to the PBS-administered group.
FIG. 42 is a plot illustrating the correlation between the rate of change in hs-CRP levels, a known NASH-related parameter, in serum ApoA-1 and exosome-derived ApoA-1, after administering BxC-V37e to MCD-diet mice, compared to the PBS-administered group.
FIG. 43 is a plot illustrating the correlation between the rate of change in ACC1 levels, a known NASH-related parameter, in serum ApoA-1 and exosome-derived ApoA-1, after administering BxC-V37e to MCD-diet mice, compared to the PBS-administered group.
FIG. 44 is a plot illustrating the correlation between the rate of change in PCNA levels, a known NASH-related parameter, in serum ApoA-1 and exosome-derived ApoA-1, after administering BxC-V37e to MCD-diet mice, compared to the PBS-administered group.
FIG. 45 is a plot illustrating the correlation between the rate of change in hs-CRP levels, a known NASH-related parameter, in serum ApoA-1 and exosome-derived ApoA-1, after administering BxC-e to MCD-diet mice, compared to the PBS-administered group.
FIG. 46 is a plot illustrating the correlation between the rate of change in Oil red O staining, a known NASH-related parameter, in serum ApoA-1 and exosome-derived ApoA-1, after administering BxC-e to MCD-diet mice, compared to the PBS-administered group.
FIG. 47 is a plot illustrating the correlation between the rate of change in AnnexinA5 levels, a known NASH-related parameter, in serum ApoA-1 and exosome-derived ApoA-1, after administering BxC-e to MCD-diet mice, compared to the PBS-administered group.

### Best Mode for Carrying Out the Invention

A composition for prognosis of nonalcoholic fatty liver disease, the composition comprising an agent for measuring a level of exosome-derived (exosomal) ApoA-1 protein in a subject that has been administered a therapeutic agent for nonalcoholic fatty liver disease.

### Mode for Carrying Out the Invention

### EXAMPLE 1: Materials and Methods

### 1-1. Animal experiment

Six-week-old C57BL/6 male wild-type mice were obtained from Koatech Co., Ltd. and fed either chow diet (n = 6) or a methionine/choline-deficient (MCD) diet (n = 15) for 12 weeks. Animal care and procedures were approved in the rodent animal facility area of Knotus Co., Ltd. (Korea; Approval Number: 19-KE-265). After the MCD diet was fed for 18 weeks, 2 nmol/kg dulaglutide (GLP-1 receptor agonist) was subcutaneously injected into mice every other day for 4 weeks, and 400 µg/head BxC-e and BxC-V37e were intravenously injected once a day, three times a week, for 4 weeks. At the end of the experiments, the mice were anesthetized, and their serum and liver tissues were collected. The following environmental conditions were maintained: temperature, 23±3°C; relative humidity, 55% ± 15%; ventilation, 10-20 air changes/hour; luminous intensity, 150-300 Lux; and a 12 h light/12 h dark cycle.

### 1-2. Cell culture

For cell maintenance, human primary hepatocytes (ScienCell, Carlsbad, CA, USA) were cultured in hepatocyte basal medium supplemented with 5% fetal bovine serum (FBS), 1% penicillin, growth supplements (ScienCell) and THP-1 monocytes (ATCC). Then, Growth supplements (ScienCell) and THP-1 monocytes (ATCC, Manassas, VA, USA) were cultured in RPMI 1640 (Gibco, Waltham, MA, USA) supplemented with 10% FBS (HyClone, Chicago, IL, USA) and 1% antibiotic/antimycotic solution (Thermo Fisher Scientific, Waltham, MA, USA). To establish a NASH in vitro model, human primary hepatocytes were treated with 100 mM FA (oleate-palmitate, 2:1 molar ratio) in 2% FBS + DMEM for 48 hours and then treated with 100 µg/mL BxC-V37e with 100 mM FA in serum-free DMEM. In addition, BxC-V37e was simultaneously treated with 500 nM thapsigargin in serum-free DMEM for 24 h. On the other hand, the THP-1 monocytes were stimulated with 200 ng/mL PMA, 100 ng/mL LPS, and 20 ng/mL IFNγ in 10% FBS + RPMI for 24 hours. Subsequently, THP-1 monocytes were treated with 100 µg/mL BxC-V37e, 100 ng/mL LPS, and 20 ng/mL IFNγ in serum-free DMEM for 24 hours. The cells from both cell lines were grown at 37°C under 5% CO2 and 95% humidified air incubator. To assess the phosphorylation levels, human primary hepatocytes and THP-1 macrophages were treated with 100 µg/mL BxC-V37e for 24 hours. Afterward, 100 mM FA or 200 ng/mL PMA, 100 ng/mL LPS, and 20 ng/mL IFNγ were added to primary hepatocytes for 30 min (phospho-AKT and phospho-AMPK) or to THP-1 macrophages for 10 min (phospho-p65).

### 1-3. Isolation and Culturing of Progenitor Cells of Induced Pluripotent Stem Cell (iPSC)-Derived Mesenchymal Stem Cell (BxC)

Induced pluripotent stem cells (iPSC) were cultured for 7 days in DMEM supplemented with 10% FBS and 10 ng/ml bFGF. From the cultured induced pluripotent stem cells, SSEA-4 (-) cells that did not express SSEA-4 (stage-specific embryonic antigen 4) protein were separated by FACS. The separated SSEA-4 (-) cells were additionally cultured for 7 days in the same medium by passage to obtain progenitor cells of induced pluripotent stem cell-derived mesenchymal stem cells according to the present disclosure. The progenitor cells of induced pluripotent stem cell-derived mesenchymal stem cells were named BxC (Brexogen stem cells).

The progenitor cells of induced pluripotent stem cell-derived mesenchymal stem cells, named BxC, were further cultured in a culture medium [high glucose DMEM (Gibco, Cat no. 11995-065), 10% Fetal bovine Serum (HyClone), 1% MEM Non-Essential Amino Acids Solution (100×) (Gibco, Cat no. 11140-050)].

### 1-4. Isolation of exosomes (BxC-e) derived from progenitor cells (BxC) of induced pluripotent stem cell-derived mesenchymal stem cells

The culture where the progenitor cells (hereinafter referred to as "BxC") of induced pluripotent stem cell-derived mesenchymal stem cells were grown was collected and centrifuged at 300×g for 10 minutes to remove the cells and cell debris. The supernatant was filtered through a 0.22-µm filter and then centrifuged at 10000×g and 4°C for 70 minutes in a high-speed centrifuge. The supernatant thus obtained was centrifuged at 100,000×g and 4°C for 90 minutes in an ultracentrifuge to obtain exosomes as a pellet. The exosomes were diluted in phosphate buffered saline (PBS) before subsequent experiments.

### 1-5. Culture and RNA-Seq analysis of pan-PPAR agonist-pretreated iMSC

iMSC (passage 4) cultured in high-glucose Dulbecco's modified Eagle's medium (Hyclone, Chicago, IL, USA) supplemented with 15% fetal bovine serum (FBS) and 1% antibiotic/antimycotic solution ((Hyclone, Chicago, IL, USA) in a T-75 flask (Eppendorf, Hamburg, Germany) at 37°C in 5% CO2 and 95% humidified air. When reaching 90% confluence, the cells were detached using TryPLE Express (Thermo Fisher Scientific) and seeded at a density of 10,000 cells/cm2 in a 4-layer Cell Factory System (Thermo Fisher Scientific). The next day, the cells were treated with 10 µM lanifibranor (Cayman, Ann Arbor, MI, USA) for 24 hours, after which the media were aspirated, and the cells were washed with Dulbecco's phosphate-buffered saline (DPBS) (HyClone).

RNA sequencing was performed using the application provided by Macrogen Inc. Hierarchical clustering was analyzed using complete linkage and Euclidean distance as a measure of similarity to present the patterns of differentially expressed transcripts, which were satisfied with |fold change|≥ 2 and p < 0.05 (independent t-test). Gene set enrichment and pathway analyses for significant gene list were performed using gProfiler (https://biit.cs.ut.ee/gprofiler/gost) and KEGG database (http://www.genome.jp/kegg/pathway).

### 1-6. Isolation of pan-PPAR agonist-pretreated iMSC exosome

Exosomes (BxC-V37e) derived from lanifibranor-pretreated iMSC were isolated as follows.

The medium for lanifibranor-pretreated iMSC was replaced with serum-free, xono-free StemPro MSC media (Gibco). After 3 days of incubation, the culture medium was harvested, centrifuged for 10 min at 300xg, and the supernatant was centrifuged for 20 minutes at 2000xg. The supernatant was centrifuged for an additional 80 minutes at 10,000xg. Thereafter, the supernatant was filtered through a 0.2 µm vacuum filter (Merck Millipore, Burlington, MA, USA). Lastly, exosomes were isolated by ultracentrifugation at 100,000xg for 80 minutes, and the pellet was subsequently washed with PBS and subjected to ultracentrifugation (Beckman Coulter, CA, USA). The exosome pellets were resuspended in PBS.

### 1-7. Cryo-transmission electron microscopy (TEM)

A 200-mesh copper grid (MiTeGen, Ithaca, NY, USA) coated with formvar/carbon film was subjected to hydrophilic treatment. The EV suspension (4 µL) was placed on a grid and blotted for 1.5 minutes at 100% humidity and 4°C. The exosomes on the grid were visualized at 36,000 x magnification using a Talos L120C FEI TEM (Thermo Fisher Scientific) at 120 kV.

### 1-8. Nanoparticle tracking analysis (NTA)

Measurements of particle size distribution and concentration of BxC-V37e were performed using a NanoSight NS300 instrument (Malvern Panalytical, Malvern, UK) based on NTA. For the analysis, BxC-V37e were diluted in sterile PBS (1:100) to reach the optimal volume for NTA. Measurements were performed at room temperature ranging from 23.0 to 25.2°C using a 488 nm Blue laser and an sCMOS camera in several repeats. Sample analysis was conducted for 10 minutes under the following camera settings and processing conditions: Shutter 600, Gain 250, camera level 10, NTA version 3.0 0064, and Detection Threshold 10.

### 1-9. Labeling of BxC-V37e with DiR and DiD and fluorescent imaging

BxC-V37e were incubated with 1 µg/mL DiR buffer for 10 minutes at 37°C according to the protocol mentioned by Lipophilic Tracers (Invitrogen, Waltham, MA, USA). Next, the DiR-labeled pan BxC-V37e were centrifuged for 80 minutes at 100,000xg and 4°C and washed with PBS (Gibco). Lastly, 200 or 400 µg of DiR-labeled BxC-V37e was resuspended in 0.1 mL of PBS and intravenously injected into C56BL/6 mice. After 24 hours, DiR-labeled BxC-V37e was detected using an In Vivo Imaging System (Caliper Life Sciences, Waltham, MA, USA) at excitation and emission wavelengths of 740 and 790 nm, respectively. The intensity of the region of interest was plotted in units of the maximum number of photons per second per centimeter square per steradian (p/s/cm2/sr). The procedure for preparing DiD-labeled BxC-V37e was identical to the procedure described above. DiD-labeled BxC-V37e were used to treat the human primary hepatocytes or THP-1 macrophages for 24 hours with or without each stimulus. After 24 hours, DiD-labeled BxC-V37e was observed under a Nikon Eclipse Ti2-U fluorescent microscope (Nikon, Tokyo, Japan).

### 1-10. Bioinformatic analysis

After treatment of HepG2 cells with 100 mM fatty acids (oleate : palmitate, 2:2) for 6 hours, total RNA was isolated using the RNeasy Mini Kit (Qiagen, Hilden, Germany). The recovered RNA was profiled using the GeneChip^{®} Human Gene 2.0 ST array (Affymetrix, Santa Clara, CA, USA). The fold change cutoff for FA-induced DEGs was set at 1.5. The DEGs were subjected to Gene Set Enrichment Analysis with KEGG collection at an FDR q-value cutoff of 0.05 (http://www.gsea-msigdb.org/gsea). The signature of BxC-V37e was constructed through transcriptomic, proteomic, and Connectivity map analyses. Briefly, FA-treated HepG2 cells were treated with BxC-V37e, and total RNA was profiled as described above. The DEGs induced by BxC-V37e was identified at a fold change cutoff of 1.5. Proteins enriched in BxC-V37e were qualitatively and quantitatively identified using LC-MS/MS (Yonsei ProteomeTech Inc., Seoul, Korea). The DEGs induced by BxC-V37e was subjected to Connectivity map analysis, and drugs with a similar transcriptome profile as that of BxC-V37e and their target genes were identified. The DEGs induced by BxC-V37e, proteins of BxC-V37e, and target genes of BxC-V37e-like drugs identified were confirmed as BxC-V37e signatures. The established signature of BxC-V37e was subjected to protein-protein interaction network and functional enrichment analyses with interaction confidence of 0.9 (https://string-db.org).

### 1-11. Flow cytometry

The isolated exosomes were stained using human MACSPlex Exosome Kit, human (Miltenyi Biotec, Bergisch Gladbach, Germany), and analyzed using an Attune NxT flow cytometer (Thermo Fisher Scientific). For analyzing the effect of exosomes on hepatocyte regeneration, the hepatocytes were stained with anti-human CD90 APC-Cy7 antibody (BioLegend, San Diego, CA, USA) after exosome treatment and analyzed using the Attune NxT flow cytometer (Thermo Fisher Scientific). To confirm whether the iMSCs express the typical cell surface markers for MSCs, the iMSCs were stained with CD73 APC, CD105 PE, CD45 FITC, CD31 PE, and CD34 APC (eBioscience, Waltham, MA, USA), and CD90 APC-Cy7 (BioLegend) antibodies. Flow cytometric analysis was conducted using an Attune NxT flow cytometer (Thermo Fisher Scientific).

### 1-12. Serum biochemical examination

Serum samples were collected 4 weeks after the initiation of BxC-V37e injection, and the following parameters were measured using a blood biochemical analyzer (7180, Hitachi, Japan): ALT (Alanine transaminase), AST (Aspartate transaminase), TG (Triglyceride), glucose, TC (total cholesterol), HDL_C (high-density lipoprotein cholesterol), LDL_C (low-density lipoprotein cholesterol), LDH (lactate dehydrogenase), and GGT (gamma-glutamyltransferase).

### 1-13. Real-time qPCR

Total RNA was isolated from liver tissues and various cell types using TRIzol^{®} (Ambion, Waltham, MA, USA). cDNA was synthesized using 1 µg of total RNA using AccuPower^{®} CycleScript RT PreMix dT20 (Bioneer, Daejeon, South Korea). Amplification was performed using the PowerSYBR^{®} Green PCR Master Mix (Applied Biosystems) according to the manufacturer's protocol. The gene expression levels were analyzed using real-time qPCR with the QuantStudio^{™} 5 Real-Time RCR System (Applied Biosystems).

GAPDH was used as the reference for normalizing the differences in the quantity of mRNA in each sample. The relative gene expression levels were analyzed using the comparative 2-ΔΔCt method. Each experiment was performed in triplicate.

### 1-14. Western blot

Cells or tissues were lysed in NP40 lysis buffer (Life Technologies, Carlsbad, CA, USA) supplemented with protease inhibitors (Thermo Fisher Scientific). The protein concentration was measured using the Bradford Assay^{™} Reagent (Thermo Fisher Scientific) according to the manufacturer's protocol. Samples were diluted at a 3:1 ratio using the 4 x Laemmli buffer (Bio-Rad Laboratories) and heated at 100°C for 10 minutes. Proteins were loaded and separated on precast polyacrylamide Mini-PROTEAN TGX gels (Bio-Rad Laboratories) and transferred to PVDF membranes (Bio-Rad Laboratories). The membranes were blocked with EveryBlot Blocking Buffer (Bio-Rad Laboratories) for 5 minutes and then treated overnight with primary antibodies at 4°C. All primary antibodies were diluted in the EveryBlot Blocking Buffer. The primary antibodies used were as follows: anti-GM130, PCNA, AMPK, phospho-AMPK (Thr172), phospho-p65 (Ser536), Pan-Akt, Phospho-Akt (Thr308) (Cell Signaling Technology, Leiden, The Netherlands), anti-CD9, Calnexin, Abca1, IL-1β, p65, ACC1, Annexin5, β-actin, GAPDH (Abcam, Cambridge, UK), anti-ApoA-1 (LSBio, Seattle, WA, USA), anti-TSG101, CD81 (Invitrogen), anti-TNF-α, PGC-1α, NRF2, and CHOP (Novusbio, Centennial, CO, USA). Western blotting for all target proteins, except CD81, was performed under reducing conditions. The membranes were washed for 10 minutes five times and then treated with the secondary antibodies for 1 hour. Anti-rabbit IgG and anti-mouse IgG (Abcam) antibodies were used as the secondary antibodies. After the membranes were washed for 10 minutes five times, the target proteins were detected using the ECL Select^{™} Western Blotting Detection Reagent (GE Healthcare, Little Chalfont, UK) and analyzed using the ChemiDoc Imaging System (Bio-Rad Laboratories).

### 1-15. Enzyme-linked immunosorbent assay (ELISA)

ELISA was performed using commercially available mouse ELISA kit. ELISA kits for insulin (Novusbio), hs-CRP (R&D systems), FFA (free fatty acids), and ApoA-1 (Abcam) were used according to the manufacturer's instructions. The assay sensitivity was < 0.19 ng/mL for insulin, < 0.015 ng/mL for hs-CRP, and 11.2 pg/mL for ApoA-1. The intra- and inter-assay coefficients of variance were < 5.93% and < 6.35% for insulin, < 7.7% and < 10.8% for hs-CRP, and < 4.7% and < 5.6% for ApoA-1, respectively.

### 1-16. Histopathological analysis

Liver tissues were fixed in 10% paraformaldehyde and subjected to general tissue treatment, such as cutting, dehydration, and paraffin embedding. The tissues were sectioned at 5 µm and mounted on the slides. The specimens were deparaffinized using xylene. The tissues were rehydrated with ethanol and stained with hematoxylin & eosin (H&E). In case of Oil red O staining, the tissues were embedded using OCT compound (Sakura Finetek, Torrance, CA, USA) and then sectioned at 20 µm using a cryotome (Leica, Wetzlar, Germany). The histopathological samples obtained were analyzed using Zen 2.3 blue edition image analyzer (Carl Zeiss, Oberkochen, Germany), and the values were normalized as the percentage of the staining area by total area. NAS examination was performed according to the histological criteria, and the levels of macrovesicular steatosis, microvesicular steatosis, and hypertrophy were scored from 0 to 3, based on the observation of the occupied area by the total area. Both steatosis and hypertrophy were evaluated at magnifications of 40x to 100x. For inflammation score, five fields were selected randomly and provided scores from 0 to 3.

### 1-17. Immunohistochemical staining

Glass slides with slices of liver tissue were placed in a drier maintained at 60°C, dried for 1 hour, and deparaffinized using xylene. The tissues were rehydrated and incubated with 0.03% peroxidase for 15 minutes to block endogenous peroxidase activity. Antigen retrieval was performed by incubating the tissue sections with Tris-EDTA buffer (pH 9.0) at 121°C for 15 minutes using a pressure cooker. To prevent non-specific reactions, 4% BSA and dextran was added for 30 min. Subsequently, the sections were incubated with the anti-TNF-α (Abcam) primary antibody for l hour and then incubated with the anti-rat IgG H&L (Abcam) secondary antibody for 30 minutes at room temperature under gentle agitation. Samples were subsequently visualized under a BX53 biological microscope (Olympus, Tokyo, Japan), and representative images were captured for analysis.

### 1-18. Reactive oxygen species (ROS) assay

Primary hepatocytes were stimulated with 400 µM H2O2 for 24 hours. Subsequently, H2O2-stimulated primary hepatocytes were treated with BxC-V37e in serum-free DMEM culture media for 24 hours, followed by washing with DPBS. Briefly, CellROX^{®} Reagent (Life Technologies) was mixed in serum-free DMEM at a final concentration of 5 µM and added to the human primary hepatocyte culture. The human primary hepatocytes were incubated for 30 minutes at 37°C. Following staining, the cells were fixed in 4% paraformaldehyde (Fujifilm Wako Chemicals, Richmond, VA, USA) for 10 minutes and then washed three times with DPBS. The nuclei and cell bodies were counterstained with NucBlue^{™} Fixed Cell stain or CellTracker^{™} (Life Technologies), respectively. After this process, all samples were observed using Nikon Eclipse Ti2-U (Nikon, Tokyo, Japan), and the percentage of ROS-positive cells was analyzed based on nuclei intensity.

### 1-19. Cell viability assay

Primary human hepatocytes (2x103 cells/mL) were seeded in 96-well plates and cultured for 4 hours in serum-free DMEM at 37°C and 5% CO2. The absorbance (OD value) was measured at 450 nm using a multiplate reader (Thermo Fisher Scientific). The effects of BxC-V37e on the viability of primary hepatocytes were evaluated using the Cell Counting Kit-8 (CCK-8) (Enzo life sciences, Farmingdale, NY, USA) according to the manufacturer's instructions.

### 1-20. Concentration of metabolic parameters and exosomal ApA-1 in human serum

Under the research protocols approved by the Institutional Review Board of Asan Medical Center, Seoul (approval number: 2008-0367) and Inha University Hospital (approval number: 08-115, 2016-06-015), metabolic parameters of serum samples were examined from 20 healthy subjects, 19 obese NAFLD subjects, and 16 obese NAFLD subjects with type 2 diabetes. The healthy group had selective abdominal surgery for benign diseases at the Department of Obstetrics and Gynecology, Asan Medical Center, Seoul. All obese groups underwent laparoscopic RYGB surgery. Subjects with malignant or severe liver or kidney disease and pregnant or breastfeeding women were excluded, and written consent was provided from all subjects at the time of enrollment. The diagnosis of NAFLD was based on clinical features and blood tests. All subjects discontinued diabetes and hypertension medications three days prior to blood collection. Blood samples were collected after a 12-hour fast, and plasma and serum were immediately separated by centrifugation. Circulating concentrations of ALT, AST, fasting glucose, insulin, HOMA-IR, cholesterol, triglycerides, Hepatic Steatosis Index (HSI), NAFLD Liver Fat Score, and hs-CRP were measured as known in the art. The concentration of exosome-derived ApoA-1 was measured using a human ELISA kit (Abcam) according to the manufacturer's protocol. The analytical sensitivity for ApoA-1 was < 3 ng/mL, and the intra- and inter-assay coefficients of variation were < 4% and < 10%, respectively.

### 1-21. Statistical analysis

Statistical analyses were performed using SPSS (version 18.0 for IBM, Chicago, IL, USA). For comparisons involving three or more groups, one-way analysis of variance (ANOVA) was used followed by Tukey's post hoc test. For comparisons involving only two groups, the paired Student's t-test was used. Data are expressed as means ± standard deviation (SD), and values with P < 0.05 were considered statistically significant.

### EXAMPLE 2: Effect of BxC-V37e Exosome on NASH

### 2-1. Characterization of BxC-V37e for NASH

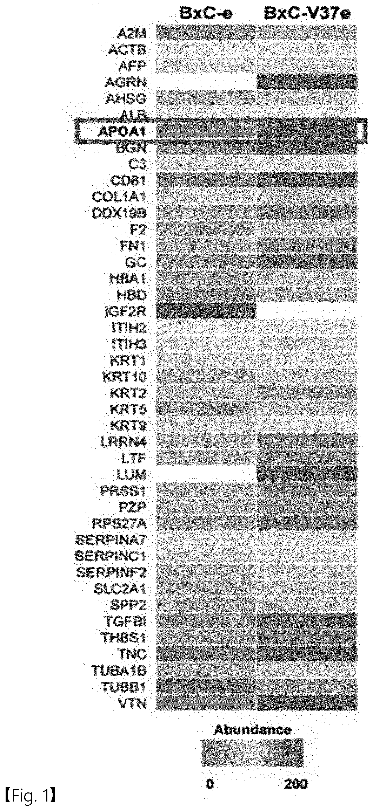

Using LC-MS/MS, a total of 32 proteins enriched in BxC-V37e were identified, which included apolipoprotein A-1 (abundance=191.3), CD81 antigen (abundance=183.8), thrombospondin-1 (abundance=155.7), collagen alpha-1 (abundance=118.1), and alpha-2-antiplasmin (abundance=77.2) (FIG. 1).

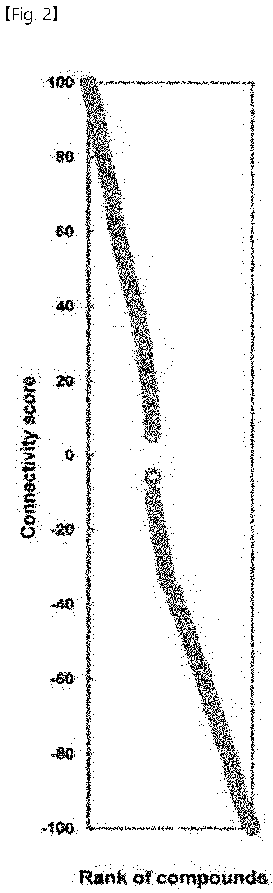

To functionally predict the pharmacological outcome of BxC-V37e, connectivity map analysis was conducted. As a result, 18 drugs were observed to be associated with 59 target genes that were significantly similar to those expressed in the transcriptome profile of BxC-V37e, and the drugs included the triciribine (AKT inhibitor, Connectivity score=99.75), EI-273 (PKC inhibitor, 99.59), 4,5-dianilinophthalimide (EGFR inhibitor, 98.63), BRD-A94297859 (XIAP inhibitor, 98.49), and GW-0742 (PPAR receptor agonist, 94.6) (FIG. 2). In addition, functional enrichment analysis revealed that 110 genes upregulated by BxC-V37e were significantly enriched in 117 canonical signaling pathways (q <0.05). Therefore, signature of BxC-V37e was constructed using 108 genes identified through proteome, transcriptome, and Connectivity map analyses.

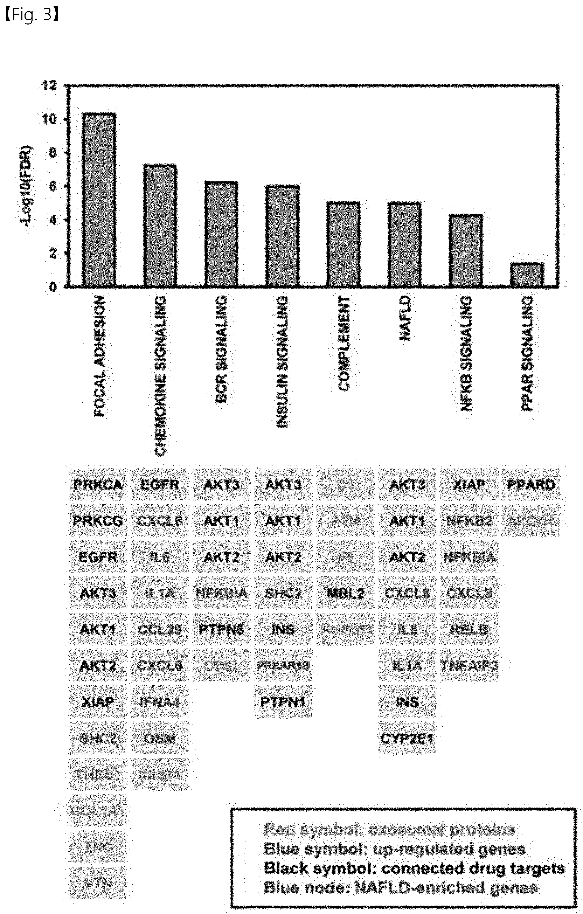

The protein-protein interaction network and functional enrichment analyses revealed that signature of BxC-V37e was significantly enriched in various signaling pathways associated with lipid metabolism, fibrosis, and inflammatory responses, including focal adhesion (q=5.7E-04), chemokine signaling pathway (q=5.5E-03), non-alcoholic fatty liver disease (q=6.2E-03), NF-kappa B signaling pathway (q=8.7E-03), insulin signaling pathway (q=1.1E-02), and PPAR signaling pathway (q=4.2E-02) (FIG. 3). These data suggest the therapeutic potential of BxC-V37e in hepatic steatosis and inflammation.

### 2-2. In vivo assessment of BxC-V37e function in NASH model

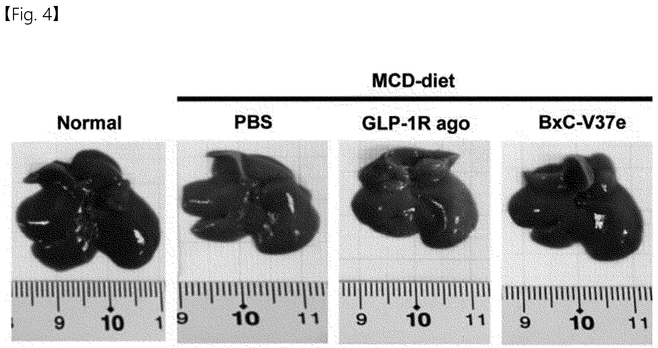

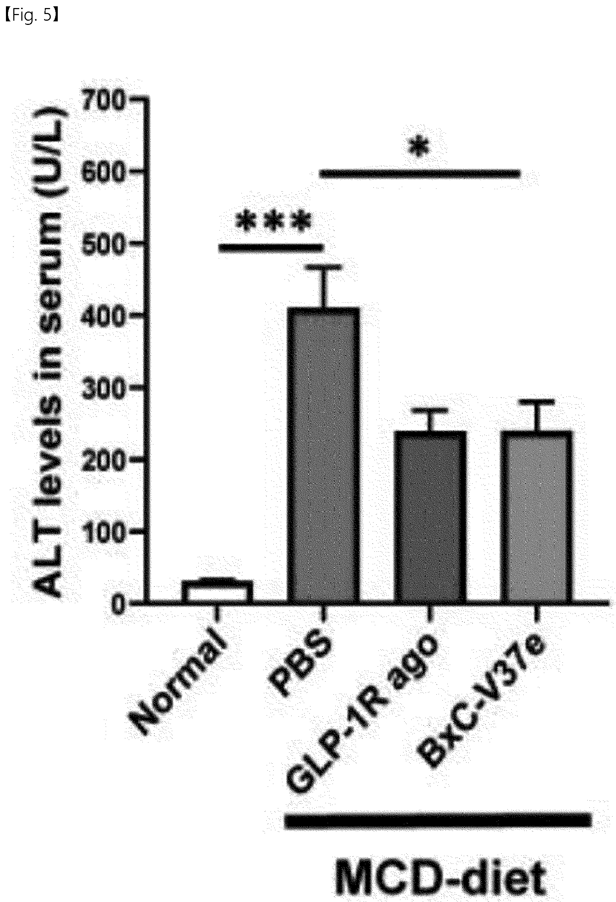

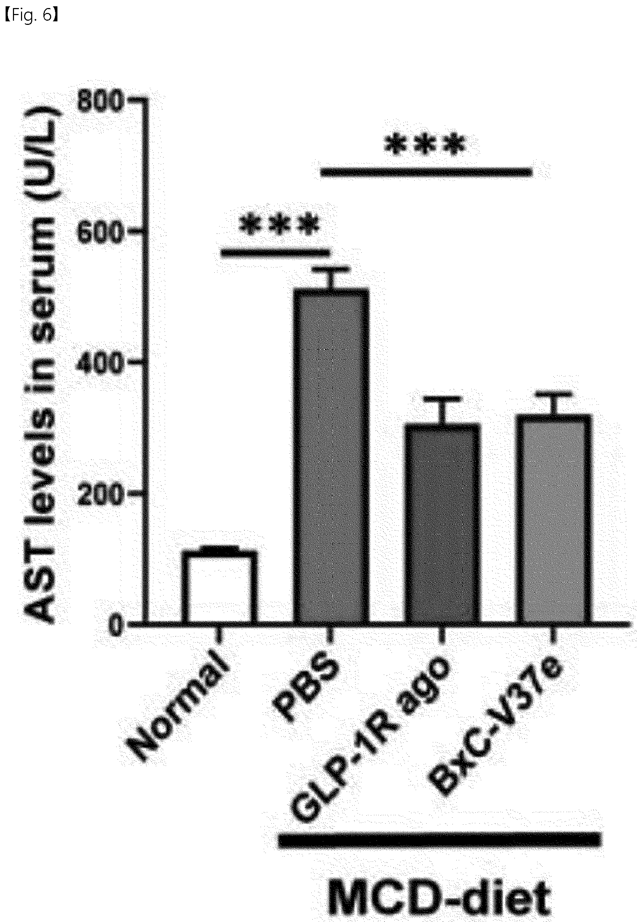

The therapeutic function of BxC-V37e was examined using a mice model of NASH induced by MC-deficient diet. With respect to the entire liver morphology, the liver of mice fed the MCD diet turned pale compared to that of normal mice (FIG. 4). In contrast, the liver of BxC-V37e-treated NASH mice was darker, similar to that observed in normal mice. No difference was observed in the livers from Dulaglutide- or PBS-treated mice. The whole liver weight did not differ between the PBS- and BxC-V37e-treated NASH mice (data not shown; MCD+PBS, 5.78±0.12% vs. MCD+BxC-V37e, 5.33±0.23%). Serum analysis revealed that the concentration of liver functional markers (ALT and AST) decreased significantly in the MCD + BxC-V37e mice compared to that in the PBS-treated mice (FIGS.5 and 6).

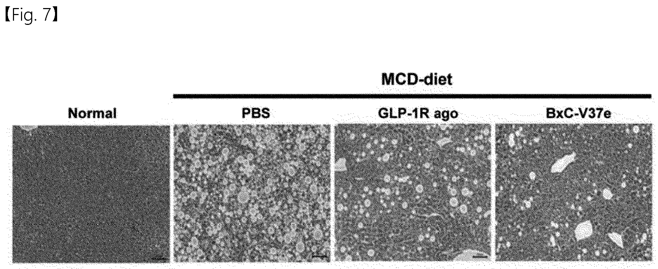

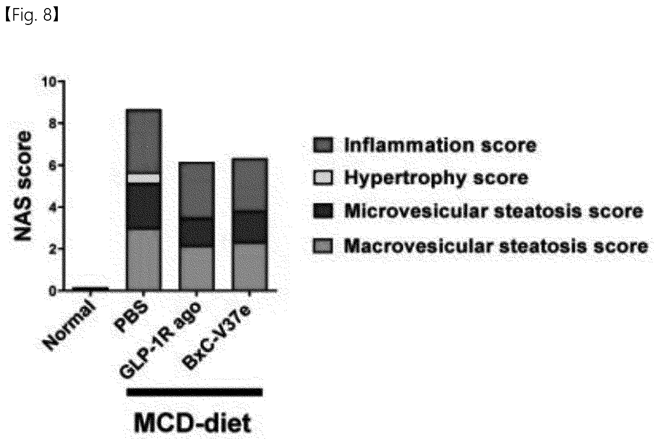

Morphological observation was made after H&E staining and treatment with BxC-V37e. a decrease in lipid droplet accumulation and inflammatory cell infiltration was detected in MCD + BxC-V37e mice compared to that in MCD+PBS mice (FIG. 7). The NAFLD activity score (NAS) was decreased in MCD + BxC-V37e mice (FIG. 8). Collectively, these data indicate that BxC-V37e can restore the liver function in NASH

### 2-3 Inhibitory effect of exosomes (BxC-e) derived from progenitor cells of induced pluripotent stem cell-derived mesenchymal stem cell on differentiation into adipocyte

Primary human adipocytes (ATCC, USA) were seeded in a 6-well plate and cultured in a 37°C, 5% CO2 incubator using DMEM media (Gibco, USA) supplemented with 1% penicillin-streptomycin and 10% CS until confluency (up to 6 days).

After culturing the adipocytes for 6 days, they were further cultured for 5 days in the basic media supplemented with adipogenic differentiation media [34µM pantothenate (Sigma), 66µM biotin (Sigma), 0.5mM insulin (Sigma), 1mM dexamethasone (Sigma), and 0.05M IBMX (Sigma)] in DMEM media (Gibco, USA) supplemented with 1% penicillin-streptomycin and 10% FBS. Thereafter, the cells were cultured for an additional 9 days in the media supplemented with adipogenic differentiation media [34µM pantothenate (Sigma), 66µM biotin (Sigma), 0.5mM insulin (Sigma), and 1mM dexamethasone (Sigma)] in DMEM media (Gibco, USA).

In this regard, the negative control was adipocytes cultured in DMEM media supplemented with 10% FBS for 14 days. A vehicle control was adipocytes that were not treated with BxC-e and cultured under adipogenic differentiation conditions for 14 days. The BxC-e-treated group included adipocytes cultured under adipogenic differentiation conditions containing BxC-e for 14 days.

After completely removing the culture medium, the cells were washed twice with PBS and fixed for 1 hour by adding 400 ul/well of 10% formalin solution. After washing with PBS, fats within the differentiated adipocytes were stained for 2 hours by adding 400 ul/well of Oil-red O working solution. Then, the Oil-red O working solution was removed, and any residue on the well walls was completely removed using secondary distilled water, after which they were dried for 5 minutes in a dryer and 500 ul/well of isopropyl alcohol was added to each well.

The amount of fat was compared by measuring absorbance at 490 nm using a microplate reader (Model 680 microplate reader, Bio-Rad, USA).

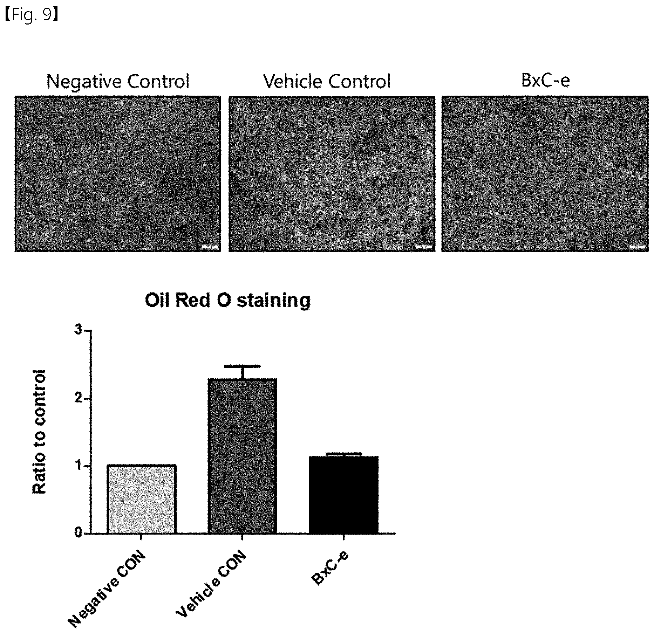

As can be seen in FIG. 9, it was found that the BxC-derived exosomes (BxC-e) of the present disclosure had an excellent effect in inhibiting lipogenesis of adipocytes.

### 2-4. Assessment for accuracy of nonalcoholic fatty liver disease diagnosis through exosomal ApoA-1 protein

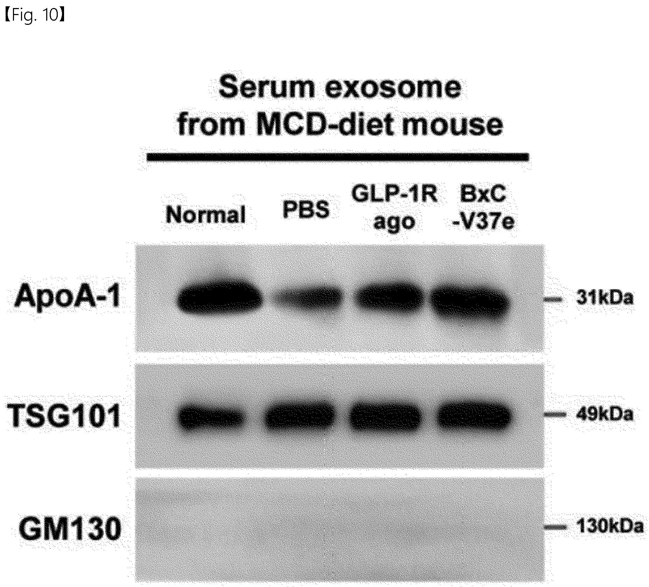

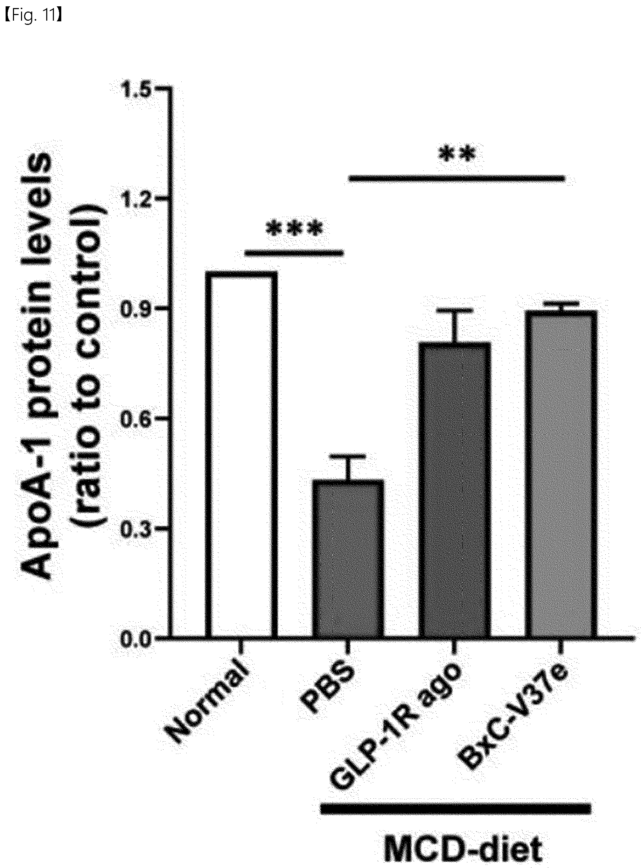

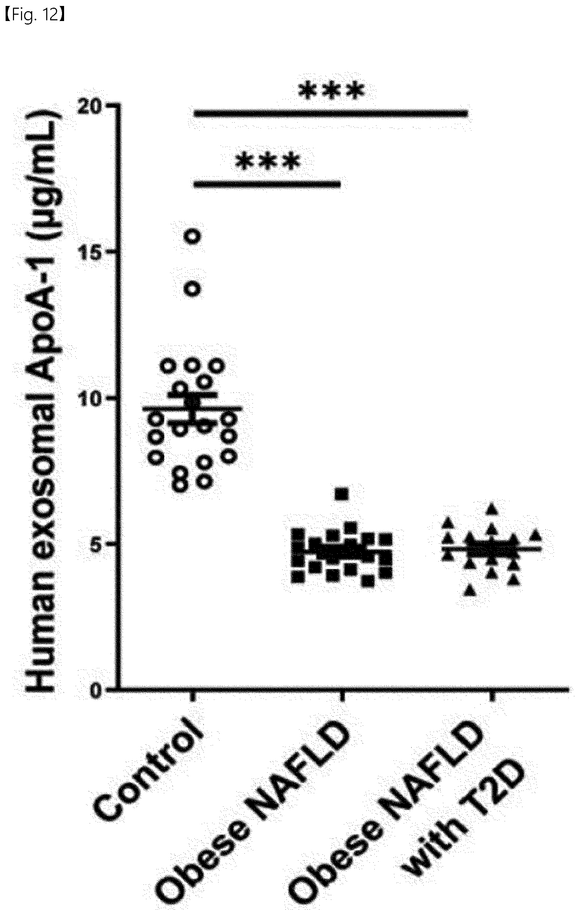

The levels of ApoA-1 protein from BxC-V37e were quantitatively compared through immunoblot analysis. The analysis showed that ApoA-1 protein derived from exosomes decreased in NASH mice while increased in animals treated with BxC-V37e (FIGS. 10 to 11). In addition, serum-derived exosomal ApoA-1 decreased in obese patients with NAFLD compared to the control group (FIG. 12).

Also, a correlation between the levels of serum ApoA-1 and exosomal ApoA-1 obtained from the ELISA assay and metabolic parameters related to nonalcoholic fatty liver disease was analyzed using the SPSS statistical program in the Spearman method. A positive R value indicates a positive correlation, a negative R value indicates a negative correlation, and if the p-value is less than 0.05, it was considered to have statistical significance.

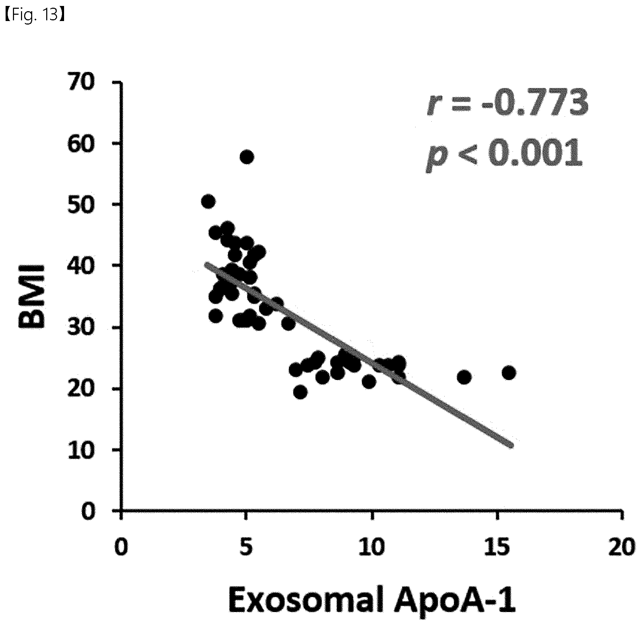

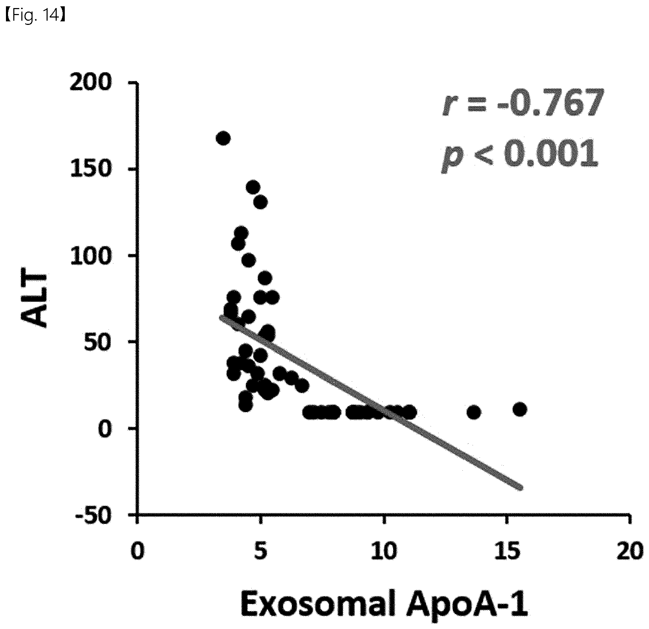

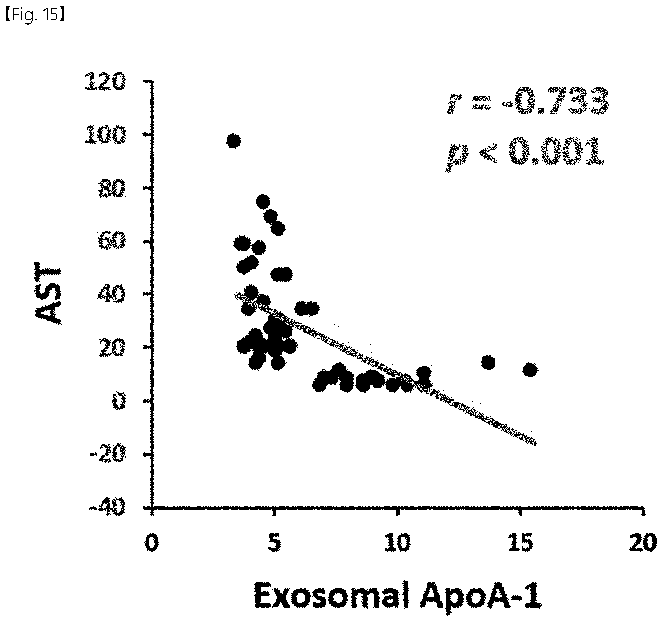

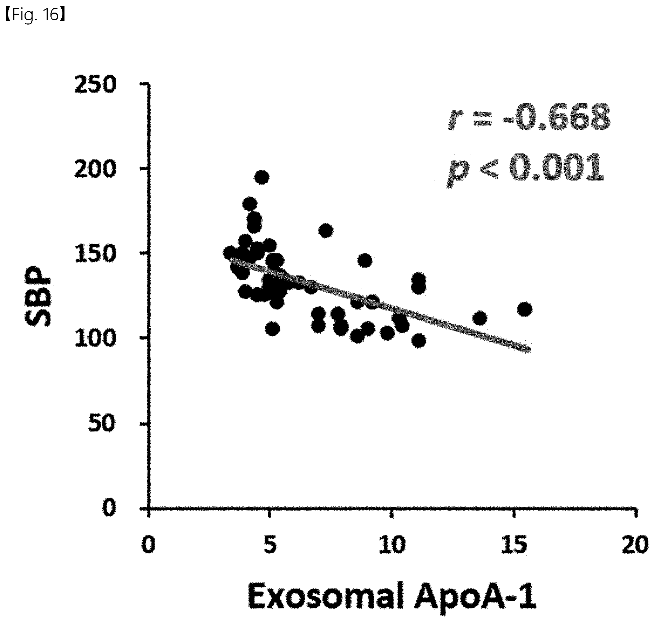

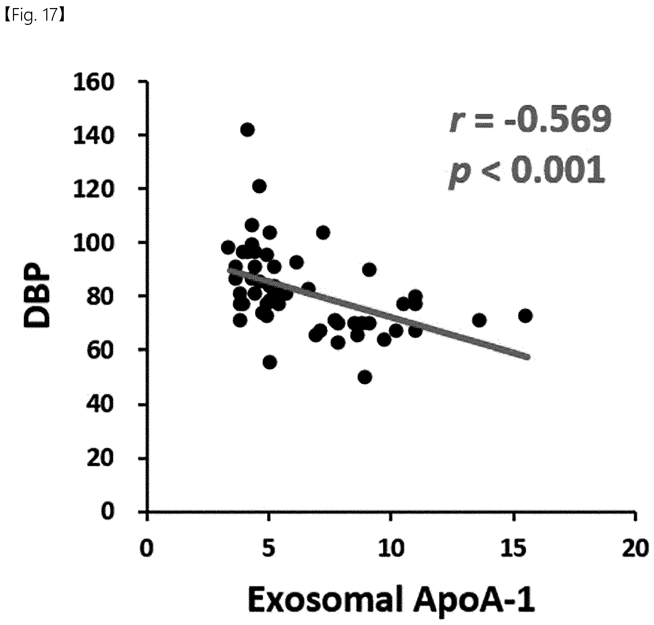

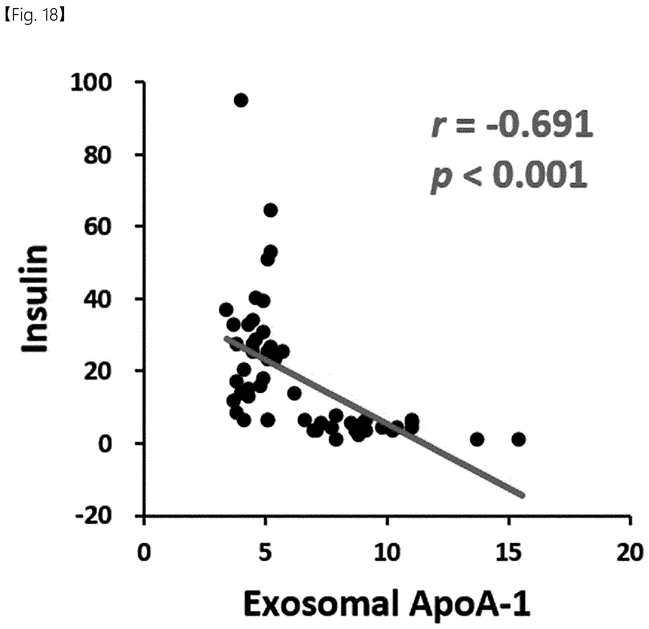

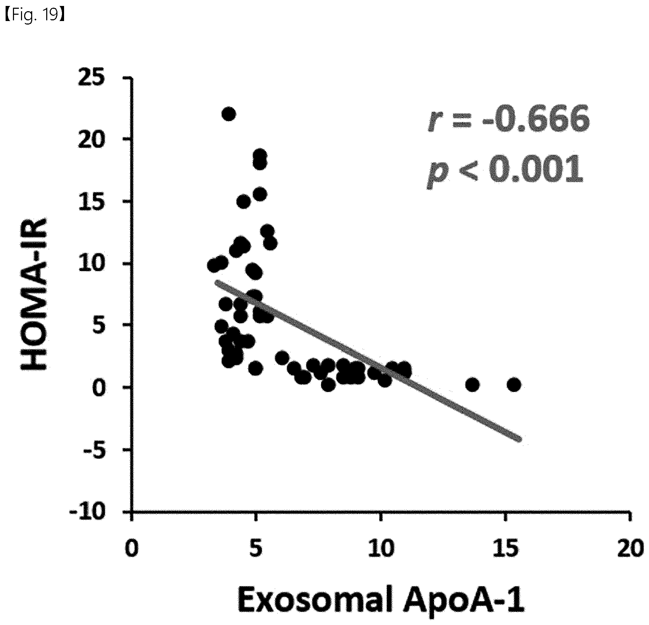

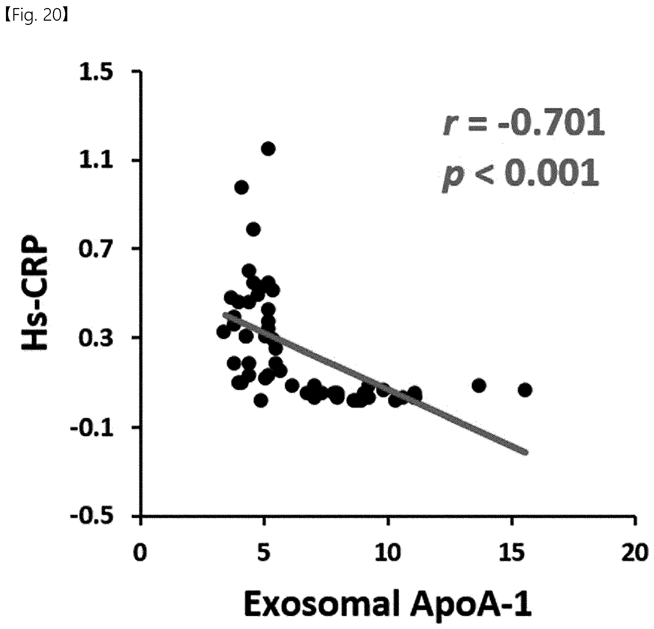

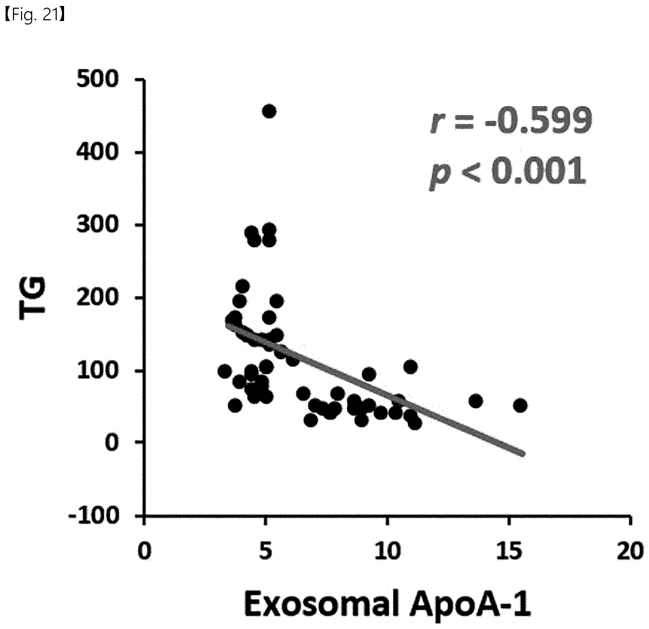

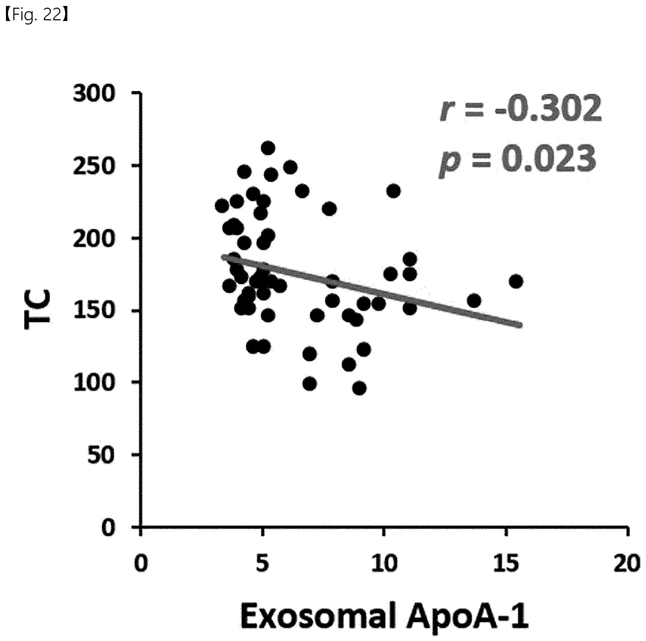

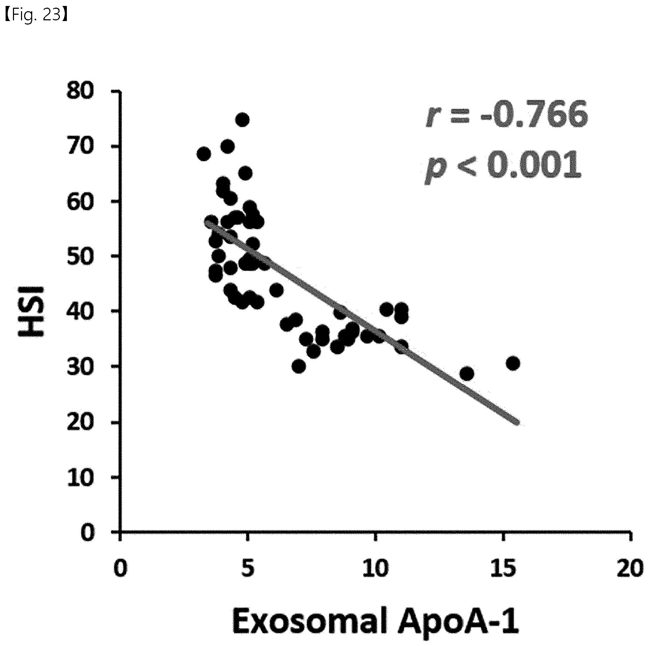

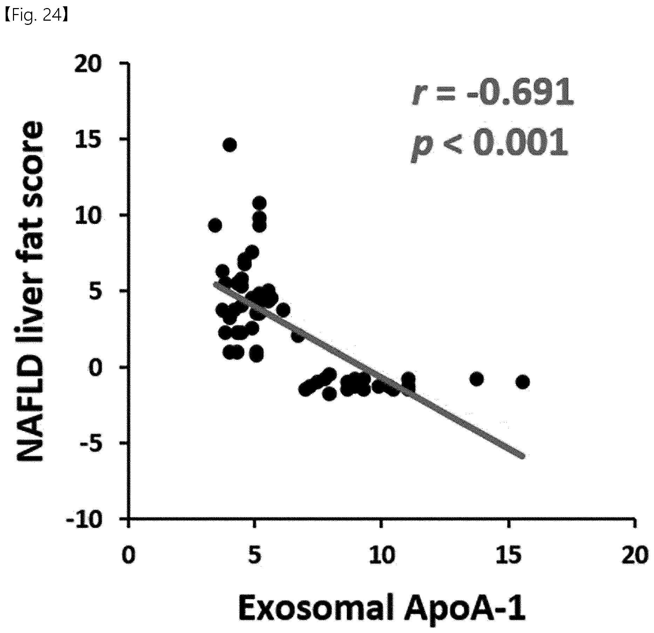

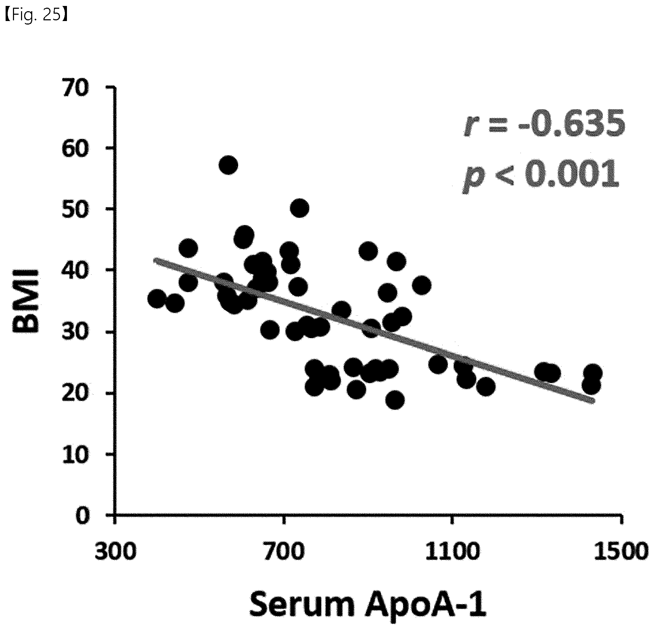

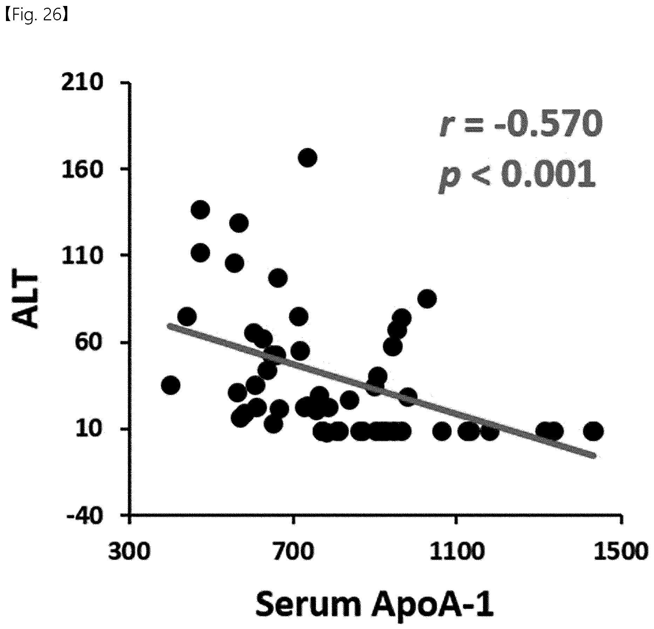

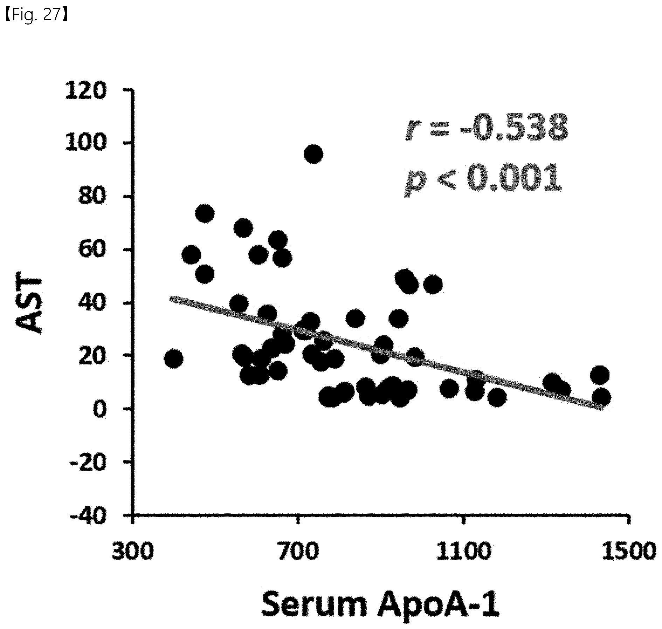

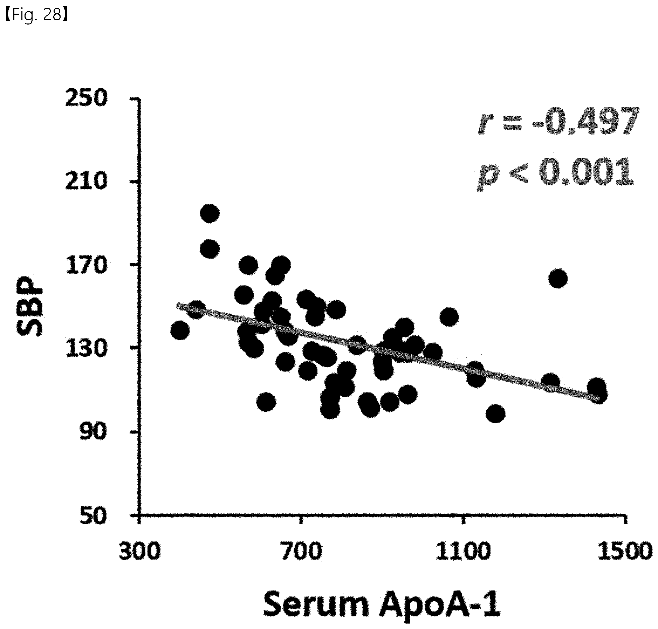

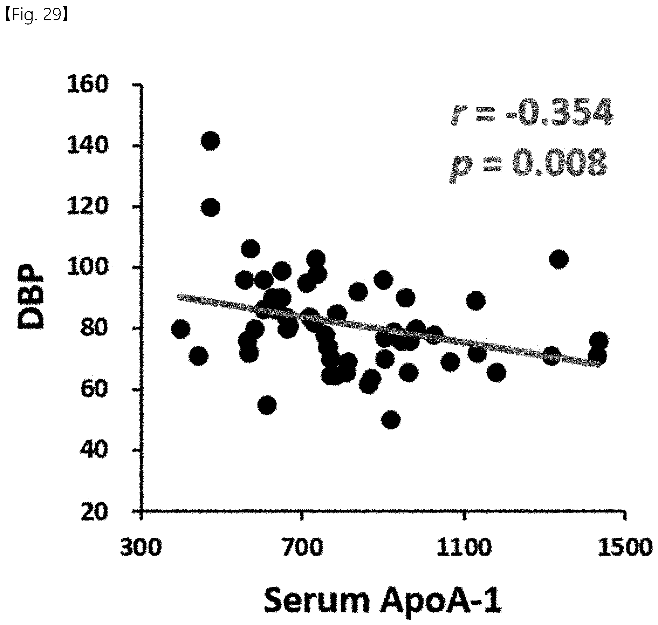

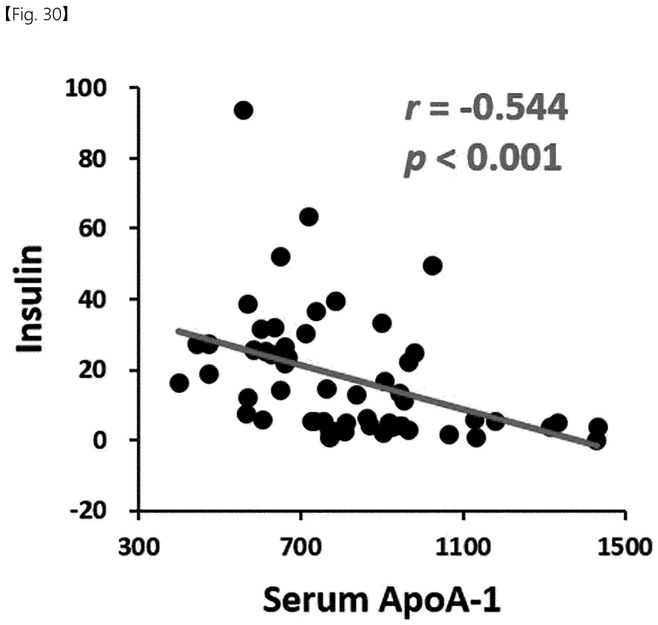

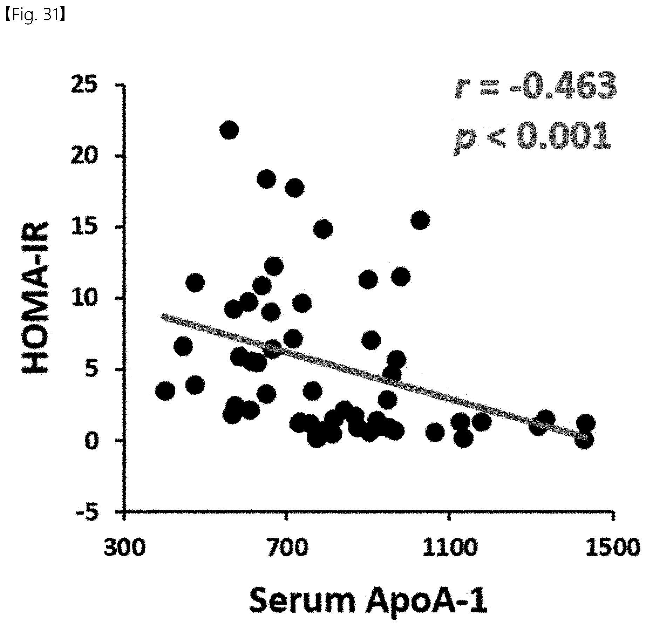

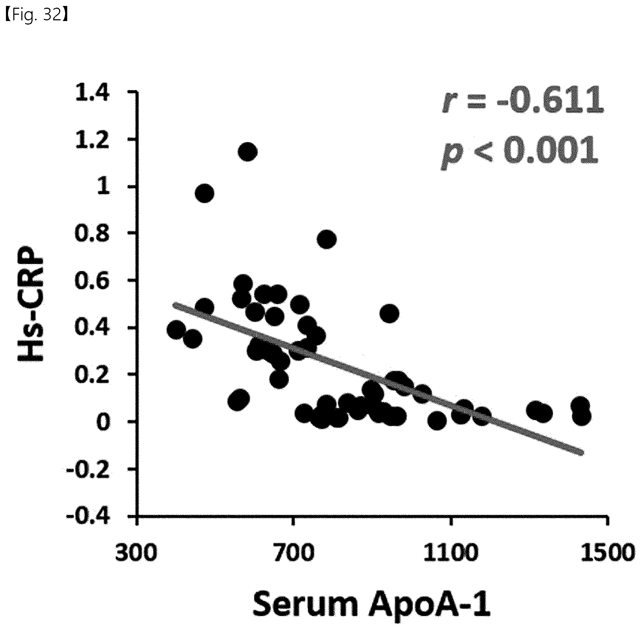

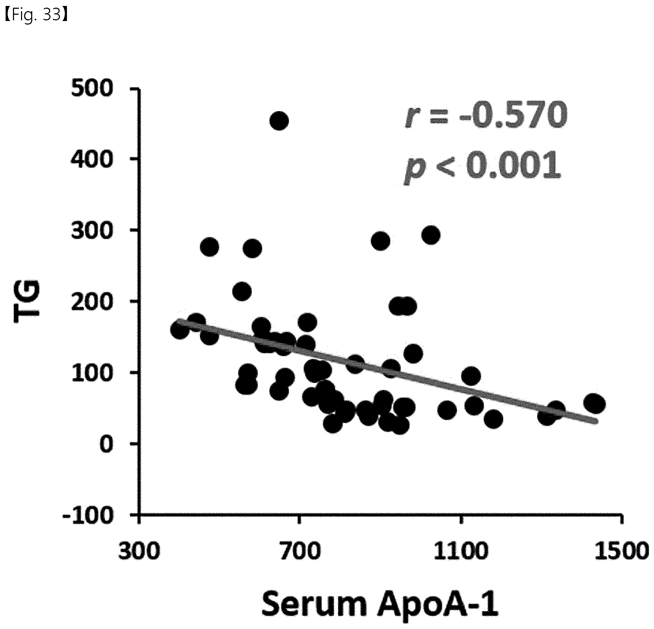

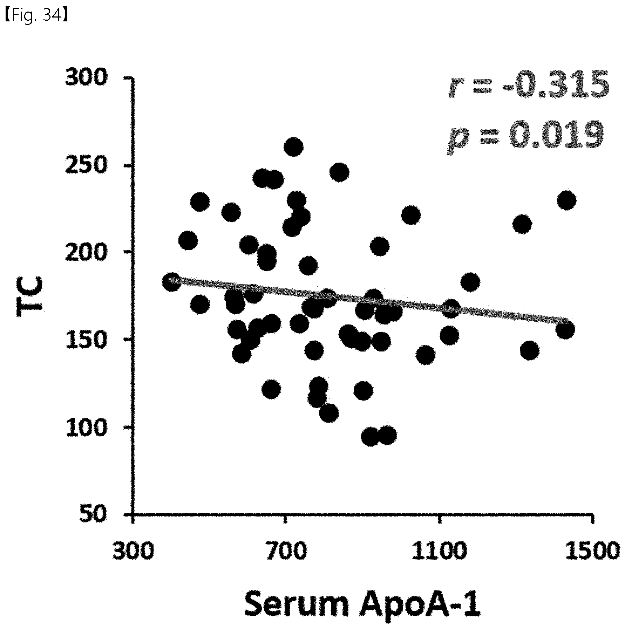

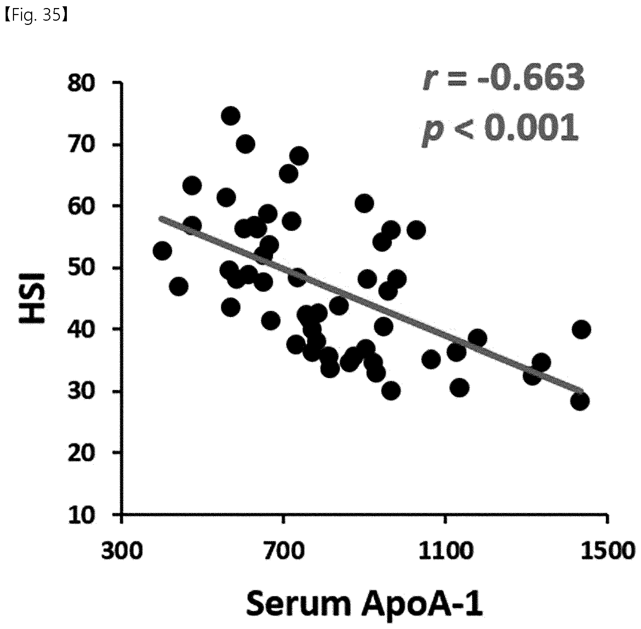

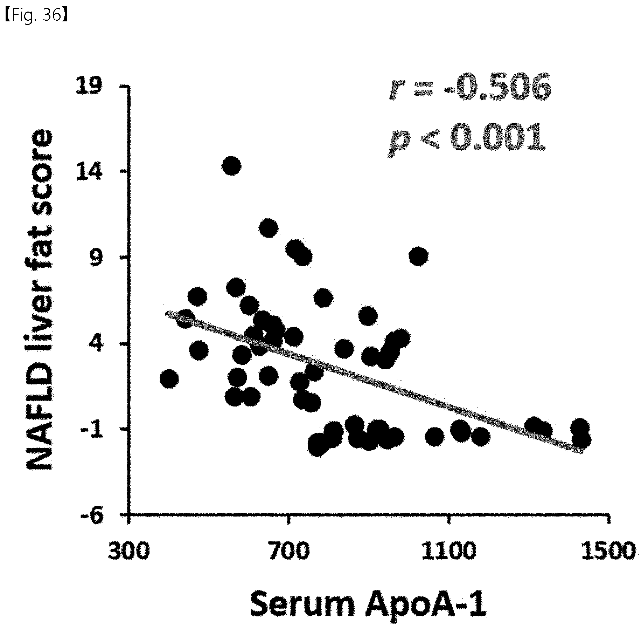

From the correlation analysis, almost all nonalcoholic fatty liver disease-related metabolic parameters showed a strong negative correlation when analyzing the correlation with ApoA-1 and metabolic parameters in human serum (FIGS. 13 to 24). Moreover, the correlation between exosomal ApoA-1 and nonalcoholic fatty liver disease-related metabolic parameters was stronger than the correlation between human serum ApoA-1 and metabolic parameters, except for the TC indicator (FIGS. 25 to 36).

These results suggest that diagnosing nonalcoholic fatty liver disease through exosome-derived ApoA-1 protein can identify the disease with far greater accuracy compared to the conventional method of diagnosing nonalcoholic fatty liver disease using ApoA-1 protein in the serum as a marker. This implies that serum-derived exosomal ApoA-1 can be used as a new biomarker for nonalcoholic fatty liver disease.

### 2-5. Assessment for accuracy of nonalcoholic fatty liver disease prognosis through exosomal ApoA-1 protein

A test was conducted to evaluate how well serum ApoA-1 and exosome-derived ApoA-1 reflect changes in established NASH-related parameters after administering NASH treatments, thereby assessing the accuracy of prognosis analysis through exosome-derived ApoA-1.

Dulaglutide, known as a NASH treatment candidate, along with BxC-e and BxC-V37e, were administered to MCD-diet mice. The associations between the rates of change in established NASH-related parameters AST, ACC1, hs-CRP, NRF2, PCNA, AnnexinA5 levels and Oil red O staining results compared to the PBS group were calculated. Statistical analysis was conducted using simple regression analysis of the SPSS statistical program (considered to have statistical significance if P value is less than 0.05).

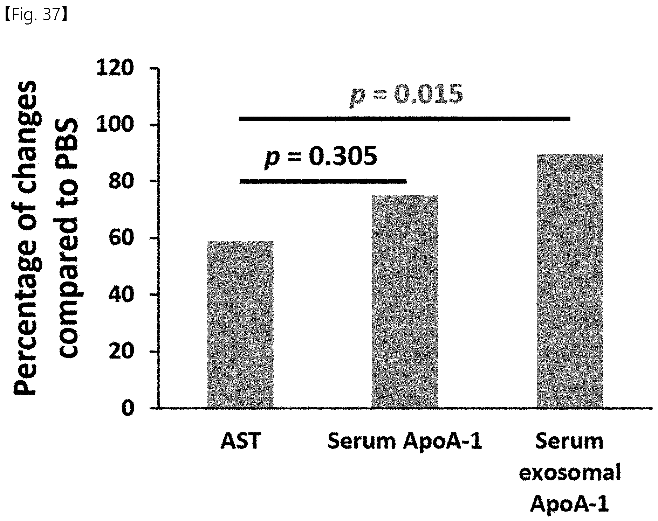

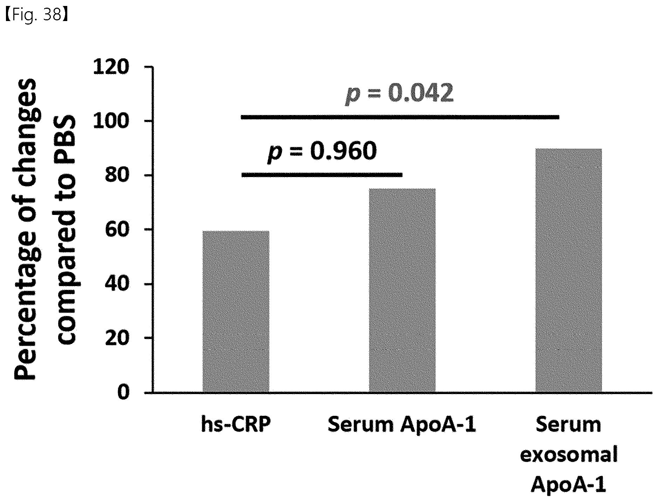

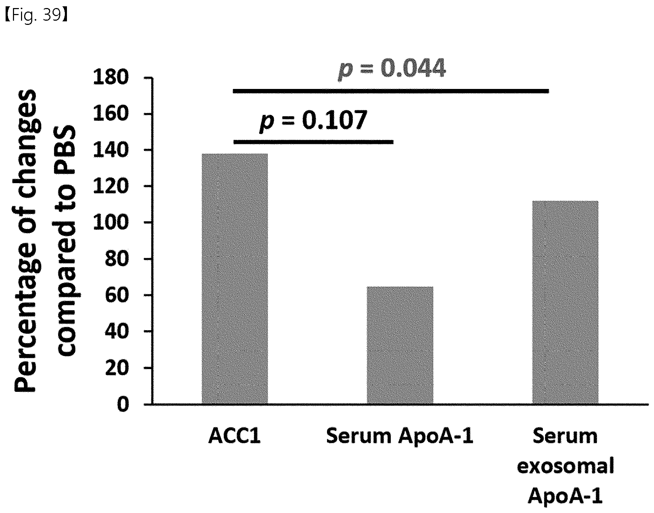

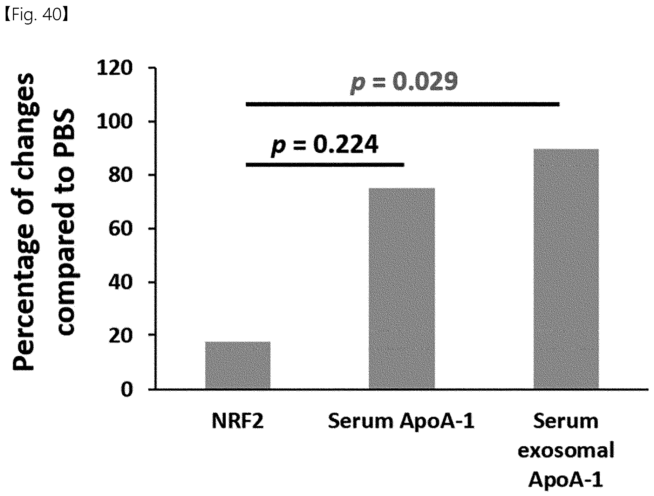

As can be seen in FIGS. 37 to 40, the rates of change in AST, ACC1, hs-CRP, and NRF2 levels in the Dulaglutide-administered group of MCD-diet mice compared to the PBS group showed a more significant correlation with the rate of change in exosome-derived ApoA-1 between the PBS group and Dulaglutide group than the rate of change in serum ApoA-1.

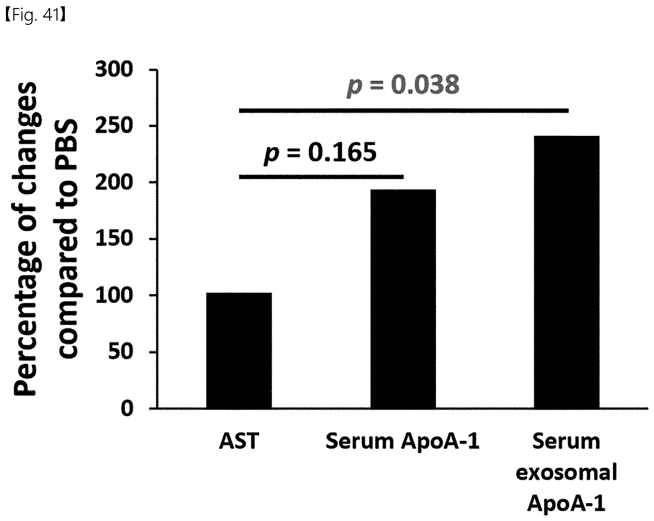

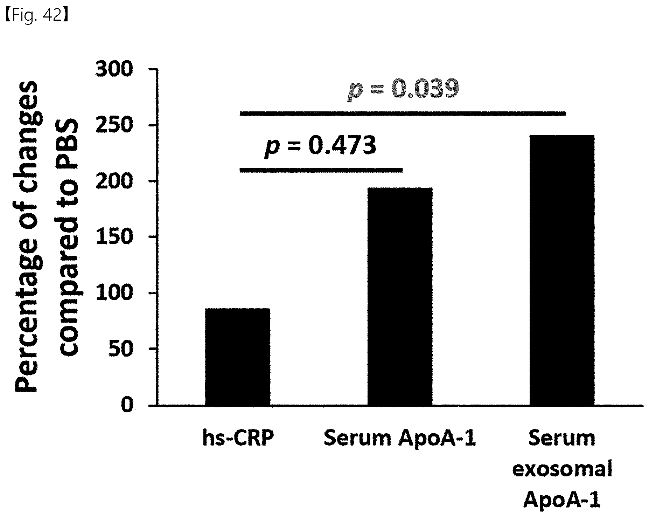

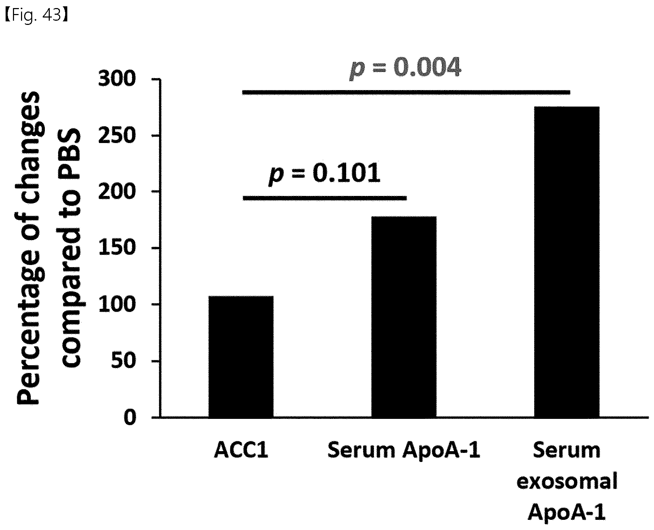

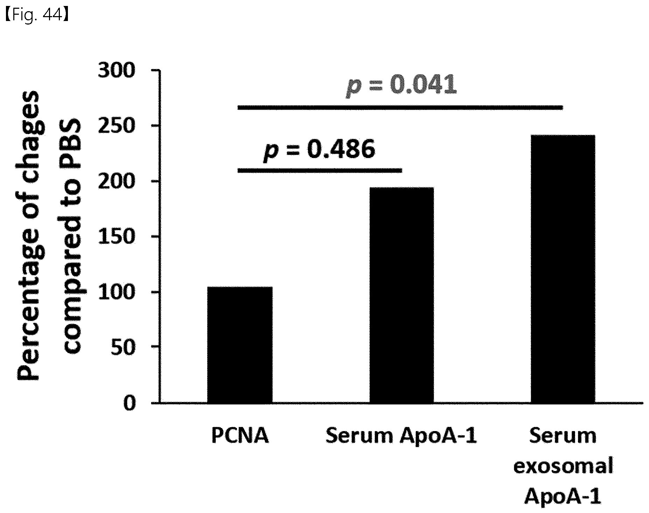

As seen in FIGS. 41 to 44, the rates of change in AST, ACC1, hs-CRP, and PCNA levels in the BxC-V37e administered group of MCD-diet mice compared to the PBS group showed a more significant correlation with the rate of change in exosome-derived ApoA-1 between the PBS group and BxC-V37e group than the rate of change in serum ApoA-1.

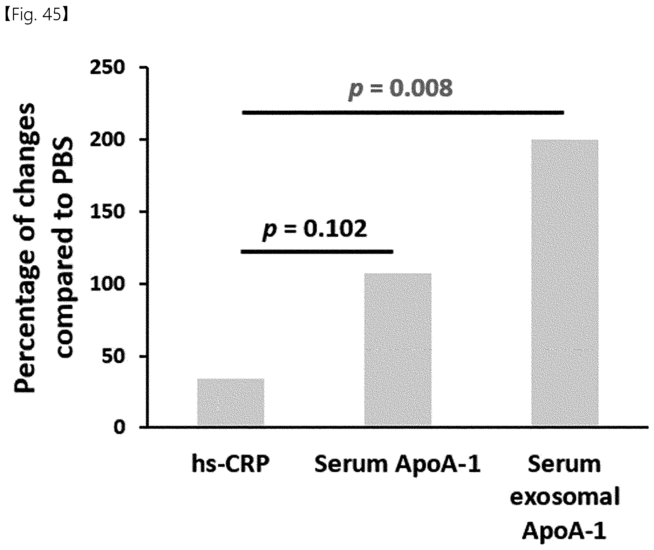

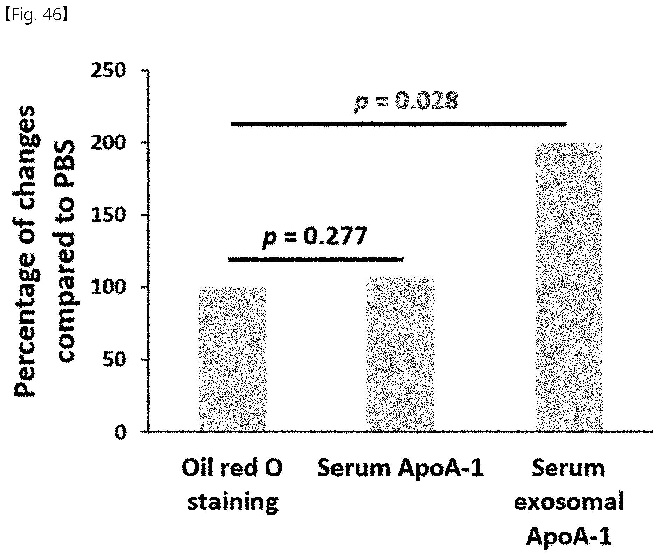

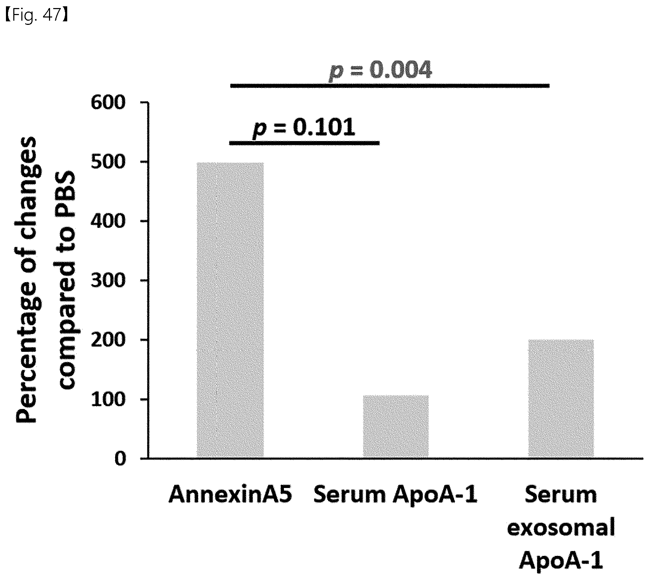

Furthermore, as shown in FIGS. 45 to 47, even when BxC-e was administered, the rates of change in hs-CRP and AnnexinA5 levels and the results of Oil red O staining in the BxC-e administered group of MCD-diet mice compared to the PBS group showed a more significant correlation with the rate of change in exosome-derived ApoA-1 than the rate of change in serum ApoA-1.

These results indicate that after administering treatments such as Dulaglutide, BxC-e, or BxC-V37e, the degree of change in liver function indicators, fat formation indicators, and inflammation indicators are more accurately reflected by exosome-derived ApoA-1 than serum ApoA-1. Thus, the nonalcoholic fatty liver disease prognosis prediction composition according to the present invention can more accurately reflect the degree of improvement in nonalcoholic fatty liver disease than existing prognosis prediction compositions or prognosis prediction methods, suggesting its versatile use in diagnosing nonalcoholic fatty liver disease.

### Industrial Applicability

The present disclosure relates to a method for providing information for diagnosis of nonalcoholic fatty liver disease (NAFLD), the method comprising a measuring step of measuring a level of exosome-derived (i.e. exosomal) ApoA-1 protein in a biological sample isolated from a subject that has developed or is suspected of onset of nonalcoholic fatty liver disease (NAFLD) therein; wherein a comparison step of comparing protein level measurements between the subject and a normal control; and wherein a determining step of determining the onset of nonalcoholic fatty liver disease in the subject when the measurement in the biological sample is lower than that in a sample from a normal control.

## Claims

1. A method for providing information for diagnosis of nonalcoholic fatty liver disease (NAFLD), the method comprising a measuring step of measuring a level of exosome-derived (i.e. exosomal) ApoA-1 protein in a biological sample isolated from a subject that has developed or is suspected of onset of nonalcoholic fatty liver disease (NAFLD) therein;
wherein a comparison step of comparing protein level measurements between the subject and a normal control; and
wherein a determining step of determining the onset of nonalcoholic fatty liver disease in the subject when the measurement in the biological sample is lower than that in a sample from a normal control.

2. The method of claim 1, wherein the measuring step comprises a contacting step of contacting the sample with an antibody or aptamer binding specifically to the protein.

3. The method of claim 1, wherein the biological sample is at least one selected from the group consisting of tissues, cells, whole blood, sera, and plasma, each isolated from a subject.

4. The method of claim 1, wherein the nonalcoholic fatty liver disease is non-alcoholic steatohepatitis (NASH).

## Patentansprüche

1. Verfahren zur Bereitstellung von Informationen für die Diagnose einer nichtalkoholischen Fettlebererkrankung (NAFLD), wobei das Verfahren einen Messschritt umfasst, bei dem der Gehalt an aus Exosomen stammendem (d. h. exosomalem) ApoA-1-Protein in einer biologischen Probe gemessen wird, die von einem Subjekt isoliert wurde, bei dem eine nichtalkoholische Fettlebererkrankung (NAFLD) aufgetreten ist oder deren Ausbruch vermutet wird;
wobei ein Vergleichsschritt zum Vergleich der Proteinmesswerte zwischen dem Subjekt und einer normalen Kontrollperson vorgesehen ist; und
wobei ein Bestimmungsschritt zum Bestimmen des Ausbruchs einer nichtalkoholischen Fettlebererkrankung bei dem Subjekt vorgesehen ist, wenn der Messwert in der biologischen Probe niedriger ist als der in einer Probe einer normalen Kontrollperson.

2. Verfahren nach Anspruch 1, wobei der Messschritt einen Kontaktierungsschritt umfasst, bei dem die Probe mit einem Antikörper oder einem Aptamer in Kontakt gebracht wird, der bzw. das spezifisch an das Protein bindet.

3. Verfahren nach Anspruch 1, wobei die biologische Probe mindestens eines aus der Gruppe bestehend aus Geweben, Zellen, Vollblut, Serum und Plasma ist, das jeweils aus einem Subjekt isoliert wurde.

4. Verfahren nach Anspruch 1, wobei es sich bei der nichtalkoholischen Fettlebererkrankung um eine nichtalkoholische Steatohepatitis (NASH) handelt.

## Revendications

1. Procédé de fourniture d'informations pour le diagnostic d'une stéatose hépatique non alcoolique (NAFLD), le procédé comprenant une étape de mesure consistant à mesurer un taux de protéine ApoA-1 dérivée d'exosomes (c'est-à-dire exosomale) dans un échantillon biologique isolé d'un sujet qui a développé ou est soupçonné d'apparition de stéatose hépatique non alcoolique (NAFLD) dans celui-ci ;
dans lequel une étape de comparaison consiste à comparer les mesures de taux de protéine entre le sujet et un témoin normal ; et
dans lequel une étape de détermination consiste à déterminer l'apparition d'une stéatose hépatique non alcoolique chez le sujet lorsque la mesure dans l'échantillon biologique est inférieure à celle dans un échantillon provenant d'un témoin normal.

2. Procédé selon la revendication 1, dans lequel l'étape de mesure comprend une étape de mise en contact consistant à mettre en contact l'échantillon avec un anticorps ou un aptamère se liant spécifiquement à la protéine.

3. Procédé selon la revendication 1, dans lequel l'échantillon biologique est au moins un sélectionné dans le groupe consistant en tissus, cellules, sang total, sérums et plasma, chacun étant isolé d'un sujet.

4. Procédé selon la revendication 1, dans lequel la stéatose hépatique non alcoolique est une stéatohépatite non alcoolique (NASH).
